# EUROPEAN PATENT APPLICATION

(11) **EP 4 603 527 A1**
(43) Date of publication of application: **20.08.2025**
(21) Application number: 25157389.5
(22) Date of filing: 12.02.2025
(51) Int. Cl.: C08J 3/12, A61K 8/02, A61Q 19/00, C08J 3/16, C08L 1/02, C08L 67/04

(54) **RESIN PARTICLES AND COSMETIC PREPARATION**

(30) Priority: 14.02.2024 JP 2024020410; 29.11.2024 JP 2024208781
(71) Applicant: Fujifilm Business Innovation Corp., Tokyo 107-0052 (JP)
(72) Inventor: ISHIZUKA, Takahiro, Minamiashigara (JP); YOSHIDA, Kazusei, Minamiashigara (JP); TAKEUCHI, Sakae, Minamiashigara (JP); YOSHIKAWA, Hideaki, Minamiashigara (JP); IWANAGA, Takeshi, Minamiashigara (JP); OKI, Masahiro, Minamiashigara (JP); MATOBA, Shota, Minamiashigara (JP); IWADATE, Yuko, Minamiashigara (JP); KASHIWAGI, Satomi, Minamiashigara (JP); AMANO, Ami, Minamiashigara (JP)
(74) Representative: Meissner Bolte Partnerschaft mbB

(57) **Abstract**

Resin particles have closed cells inside and have a volume-average particle size of 1 µm or more and 30 µm or less, the closed cells have an average long axis length of 0.1 µm or more and 2.0 µm or less, and a closed cell porosity is 1% or more and 40% or less.

## Description

### Background

### (i) Technical Field

The present disclosure relates to resin particles and a cosmetic preparation.

### (ii) Related Art

Japanese Unexamined Patent Application Publication No. 2019-218307 proposes a composition containing an aqueous medium, a lipophilic porous powder dispersed in the aqueous medium, an oil having a viscosity of 100 mPa·s or less, a dispersant that disperses the lipophilic porous powder in the aqueous medium, and hydrated particles.

Japanese Unexamined Patent Application Publication No. 2022-176884 proposes a method for improving the efficiency of extracting light from inside of the skin, the method involving applying a cosmetic composition containing particles having particle sizes in the Mie scattering region to a skin surface.

### Summary

Accordingly, it is an object of the present disclosure to provide resin particles that have closed cells inside, that have excellent shield maintaining properties in a wet state, and that can achieve both transmitting properties and haze compared to when the volume-average particle size is less than 1 µm or more than 30 µm, the average long axis length of the closed cells is less than 0.1 µm or more than 2.0 µm, or the closed cell porosity is less than 1% or more than 40%.

According to a first aspect of the present disclosure, there is provided resin particles having closed cells inside, in which the resin particles have a volume-average particle size of 1 µm or more and 30 µm or less, the closed cells have an average long axis length of 0.1 µm or more and 2.0 µm or less, and a closed cell porosity is 1% or more and 40% or less.

According to a second aspect of the present disclosure, there is provided the resin particles according to the first aspect, containing a biodegradable resin as a main component.

According to a third aspect of the present disclosure, there is provided the resin particles according to the second aspect, in which the biodegradable resin is a cellulose.

According to a fourth aspect of the present disclosure, there is provided the resin particles according to any one of the first to third aspects, in which the closed cells have an average long axis length of 0.1 µm or more and 1.5 µm or less.

According to a fifth aspect of the present disclosure, there is provided the resin particles according to any one of the first to fourth aspects, in which the closed cell porosity is 1% or more and 20% or less.

According to a sixth aspect of the present disclosure, there is provided a cosmetic preparation containing the resin particles according to any one of the first to fifth aspects.

The first aspect of the present disclosure provides resin particles that have closed cells inside, that have excellent shield maintaining properties in a wet state, and that can achieve both transmitting properties and haze compared to when the volume-average particle size is less than 1 µm or more than 30 µm, the average long axis length of the closed cells is less than 0.1 µm or more than 2.0 µm, or the closed cell porosity is less than 1% or more than 40%.

The second aspect of the present disclosure provides resin particles that have excellent biodegradability and shield maintaining properties in a wet state and that can achieve both transmitting properties and haze compared to when a non-biodegradable resin is contained as a main component.

The third aspect of the present disclosure provides resin particles that have excellent biodegradability and shield maintaining properties in a wet state and that can achieve both transmitting properties and haze compared to when a cellulose derivative is contained.

The fourth aspect of the present disclosure provides resin particles that have excellent shield maintaining properties in a wet state and that can achieve both transmitting properties and haze compared to when the closed cells have an average long axis length of less than 0.1 µm or more than 1.5 µm.

The fifth aspect of the present disclosure provides resin particles that have excellent shield maintaining properties in a wet state and that can achieve both transmitting properties and haze compared to when the closed cell porosity is less than 1% or more than 20%.

The sixth aspect of the present disclosure provides a cosmetic preparation that reduces color darkening caused by sebum wetting and exhibits an excellent soft focus effect compared to when resin particles that have closed cells inside, that have a volume-average particle size of less than 1 µm or more than 30 µm, that have a closed cell average long axis length of less than 0.1 µm or more than 2.0 µm, and that have a closed cell porosity of less than 1% or more than 40% are used.

### Detailed Description

One exemplary embodiment of the present disclosure will now be described. The descriptions and examples below merely illustrate exemplary embodiments and do not limit the scope of the present disclosure.

In any stepwise numerical range recited in the present description, the upper limit or the lower limit of one numerical range may be substituted with the upper limit or the lower limit of a different stepwise numerical range. In any numerical range recited in the present description, the upper limit or the lower limit of that numerical range may be substituted with any value disclosed in Examples.

Each of the components may contain multiple corresponding substances.

When the amount of a component in a composition is described and when there are two or more substances that correspond to that component in the composition, the amount is the total amount of the two or more substances in the composition unless otherwise noted.

### Resin particles

Resin particles according to an exemplary embodiment have closed cells inside and have a volume-average particle size of 1 µm or more and 30 µm or less, in which the closed cells have an average long axis length of 0.1 µm or more and 2.0 µm or less, and the closed cell porosity is 1% or more and 40% or less.

The resin particles according to the exemplary embodiment exhibit excellent shield maintaining properties in a wet state and can achieve both transmitting properties and haze due to these features. The reason for this is presumably as follows.

Resin particles have various usages. Thus, resin particles are required to have various functions. Some of the functions are the shield maintaining properties in a wet state and achievement of both transmitting properties and haze.

For example, a cosmetic preparation, which is one of the usages of the resin particles, is expected to have sebum absorbing properties to inhibit makeup separation, and have shielding properties to make lines, pores, etc., less noticeable. To yield these properties, it is common practice to add an inorganic powder, such as titanium oxide or zinc oxide, having a high refractive index (in other words, high-shielding power) to cosmetic preparations.

Meanwhile, recent years have seen rising bare skin trends particularly among young people, and the soft focus effect that brings moderate shielding properties while keeping some degree of transparency of bare skin is sought after. In addition, reduction of color darkening despite absorption of sebum is also desirable.

These desirable properties can be realized by using resin particles that have shield maintaining properties in a wet state and satisfy both transmitting properties and haze. In other words, since both transmitting properties and haze can be achieved, the soft focus effect that yields moderate shielding properties can be achieved, and since the shield maintaining properties is exhibited in a wet state, moderate shielding properties are maintained despite sebum absorption, and thus the color darkening can be reduced.

There are known a technology that does not use an inorganic powder having high refractive index (in other words, high shielding power) but uses hydrated particles (for example, Japanese Unexamined Patent Application Publication No. 2019-218307) and a technology that uses particles having a particle size in the Mie scattering region (for example, Japanese Unexamined Patent Application Publication No. 2022-176884); however, these technologies do not offer sufficient shield maintaining properties in a wet state and do not satisfactorily achieve both transmitting properties and haze.

As such, the resin particles desirably have improved shield maintaining properties in a wet state and achieve both improved transmitting properties and improved haze.

To address this, the resin particles according to an exemplary embodiment have closed cells inside, and the average long axis length of the closed cells and the closed cell porosity are set to be within the aforementioned ranges.

Since the closed cells of an appropriate size and in an appropriate amount are present inside the particles, light is scattered by the closed cells even when the resin particles are wet, and thus the shield maintaining properties in a wet state are improved. In addition, the blurring effect is obtained while exhibiting a transmittance.

Since the volume-average particle size of the resin particles is within the aforementioned range, the shield maintaining properties in a wet state and achievement of both the transmitting properties and haze can be more easily exhibited.

It is assumed from the matters described above that the resin particles according to the exemplary embodiment can exhibit excellent shield maintaining properties in a wet state and can achieve both transmitting properties and haze.

Furthermore, since the resin particles of the exemplary embodiment have the aforementioned properties, a cosmetic preparation that contains the resin particles of the exemplary embodiment reduces color darkening caused by sebum wetting and exhibits an excellent soft focus effect.

### Volume-average particle size

The volume-average particle size of the resin particles according to an exemplary embodiment is 1 µm or more and 30 µm or less, more preferably 2 µm or more and 20 µm or less, and yet more preferably 3 µm or more and 10 µm or less.

When the volume-average particle size of the resin particles is less than 1 µm, there is a tendency in which the closed cell porosity decreases, and it becomes difficult to attain the shield maintaining properties in a wet state and achieve both the transmitting properties and haze.

When the volume-average particle size of the resin particles is more than 30 µm, the haze increases, and it becomes difficult to achieve both the transmitting properties and the haze.

In particular, when the volume-average particle size of the resin particles is within the aforementioned range and the resin particles are used in a cosmetic preparation, cosmetic properties such as texture are improved.

The volume-average particle size of the resin particles is measured as follows.

Particle sizes are measured with an LS particle size distribution meter "Beckman Coulter LS 13 320 (produced by Beckman Coulter, Inc.)", the particle size cumulative distribution is plotted on a mass basis from the small diameter side, and the particle size at 50% is determined as the volume-average particle size.

### Average long axis length of closed cells and closed cell porosity

The resin particles according to an exemplary embodiment have closed cells inside. Here, a close cell means a cell confined by a wall.

The average long axis length of the closed cells is 0.1 µm or more and 2.0 µm or less, preferably 0.1 µm or more and 1.5 µm or less, more preferably 0.1 µm or more and 1.0 µm or less, and yet more preferably 0.2 µm or more and 0.8 µm or less.

In addition, the closed cell porosity is 1% or more and 40% or less, preferably 1% or more and 20% or less, more preferably 1% or more and 15% or less, and yet more preferably 3% or more and 10% or less.

When the average long axis length of the closed cells and the closed cell porosity are low, light scattering caused by the closed cells is inhibited, and it becomes difficult to achieve both transmitting properties and haze.

When the average long axis length of the closed cells and the closed cell porosity are high, transmitting properties are excessively enhanced, and it becomes difficult to achieve both transmitting properties and haze.

### Area ratio of open cells

The open cell porosity in the resin particles of the exemplary embodiment may be low or 0% from the viewpoint of the shield maintaining properties in a wet state. Here, an open cell is a cell that is not confined by a wall and that opens to the outside of the resin particle (when the resin particle has a coating layer described below, the outside of the base particle).

Specifically, the open cell porosity is preferably 0% or more and 10% or less and more preferably 0% or more and 5% or less.

### Confirmation of closed cells and open cells and methods for measuring average long axis length of closed cells, closed cell porosity, and open cell porosity

Confirmation of closed cells and open cells and the methods for measuring the average long axis length of closed cells, the closed cell porosity, and the open cell porosity are as follows.

Resin particles are embedded in an epoxy resin and cut with a diamond knife or the like to prepare a specimen having an observation surface, which is a surface that includes cross sections of the resin particles.

The prepared specimen is loaded onto a scanning electron microscope (SEM) to obtain a SEM image (accelerating voltage: 1.0 kV, magnification: 20,000x) of cross sections of the resin particles imaged with the scanning electron microscope.

From the obtained SEM image, the presence of the closed cells confined in walls and the open cells that extend from the resin particle surfaces (when resin particles have a coating layer described below, from the base particle surfaces) to the inside of the particles at the resin particle cross sections are identified.

Next, the obtained SEM image is binarized by image analysis software (for example, ImageJ, WinROOF) with a threshold set to clearly distinguish voids from the epoxy resin constituting the background.

Next, the longest axis length of each of closed cells in one resin particle is measured. This operation is performed on ten resin particles, and the average value of the longest axis lengths of the closed cells is obtained as the average long axis length.

Next, the area ratio of all cells relative to the entire cross section of the resin particle is determined. Meanwhile, the area ratio of closed cells relative to the entire cross section of the resin particle is determined. For the closed cells, the area ratio may be calculated without binarizing if the closed cells are directly identifiable.

The area ratio of the open cells is calculated from the equation: area ratio of open cells = area ratio of all cells - area ratio of closed cells.

This operation is performed ten times, and the obtained values are arithmetically averaged.

Here, cross sections of resin particles to be observed are selected from the cross sections of resin particles that have a cross sectional size equal to or more than 85% of the volume-average particle size. Here, the cross sectional size refers to the length of the longest straight line drawn between arbitrarily selected two points on the contour of the resin particle cross section (in other words, the long axis).

### Average circularity

The average circularity of the resin particles of the exemplary embodiment is preferably 0.88 or more, more preferably 0.90 or more, and yet more preferably 0.92 or more from the viewpoints of improving the shield maintaining properties in a wet state and improving both the transmitting properties and the haze. In particular, when the resin particles are used in a cosmetic preparation, a higher average circularity improves the texture.

The average circularity of the resin particles is ideally 1.

The circularity of a resin particle is determined by (equivalent circle perimeter)/(perimeter) [(perimeter of a circle having the same projection area as the particle image)/(perimeter of a particle projection image)]. Specifically, the circularity is measured by the following method.

First, resin particles to be measured are sampled by suction and are allowed to form a flat flow; and then particle images are captured as still images by instantaneous strobe light emission and analyzed with a flow-type particle image analyzer (FPIA-3000 produced by Sysmex Corporation) to determine the circularity. The number of particles sampled for determining the circularity is set to 3,500, and the arithmetic average is calculated and assumed as the average circularity.

### Components in resin particles

The resin particles of the exemplary embodiment contain a resin as a main component.

The meaning of "containing a resin as a main component" is that the resin content relative to resin particles (or base particles when coating layers described below are included) is 90 mass% or more (preferably 95 mass% or more, 98% or more, or 100 mass%).

From the viewpoint of imparting biodegradability, the resin may be a biodegradable resin. The biodegradable resin is a resin that is degraded by microorganisms into water and carbon dioxide. Specifically, although there is no quantitative definitions for the period and decomposition rate of biodegradable resins, a resin that has a biodegradation rate of 60% or more in 60 days is defined as a biodegradable resin according to JIS K 6950:2000 ((ISO14851:1999)

Examples of the biodegradable resin include cellulose, cellulose derivatives, polyester resins, natural polymers, and polyvinyl alcohol.

Among these, cellulose and cellulose derivatives are preferred, and cellulose is more preferred from the viewpoint of biodegradability.

### Cellulose

The number-average molecular weight of cellulose is preferably 37000 or more and more preferably 45000 or more.

There is no upper limit for the number-average molecular weight of cellulose, and the upper limit may be, for example, 100,000 or less.

The number-average molecular weight of cellulose is measured by gel permeation chromatography (differential refractometer Optilab T-rEX produced by Wyatt Technology Corporation, a multi angle light scattering detector DAWN HELEOS II produced by Wyatt Technology Corporation, columns: TSKgel α-M and α-3000, one each, produced by TOSOH CORPORATION) with dimethylacetamide (0.1 M lithium chloride added) serving as a solvent.

### Cellulose derivative

Examples of the cellulose derivative include cellulose acylate, cellulose ether, hydroxyalkyl cellulose, and carboxymethyl cellulose.

Among these, cellulose acylate is preferable as the cellulose derivative. Cellulose acylate tends to increase the rolling property, the oil absorbing property, and the mechanical strength.

Cellulose acylate is a cellulose derivative obtained by substituting at least one of the hydroxy groups in cellulose with an acyl group (acylation). An acyl group is a group having a -CO-R^{AC} (R^{AC} represents a hydrogen atom or a hydrocarbon group) structure.

Cellulose acylate is, for example, a cellulose derivative represented by general formula (CA) below.

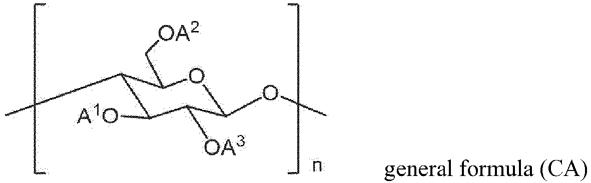

In general formula (CA), A¹, A², and A³ each independently represent a hydrogen atom or an acyl group, and n represents an integer of 2 or more. Here, at least one selected from n A¹, n A², and n A³ represents an acyl group. In the molecule, n A¹ may be the same or partly the same, or different from one another. The same applies to n A² and n A³ in the molecule.

The acyl group represented by A¹, A², and A³ may have a linear, branched, or cyclic hydrocarbon group, preferably has a linear or branched hydrocarbon group, and more preferably has a linear hydrocarbon group.

The acyl group represented by A¹, A², and A³ may have a saturated or unsaturated hydrocarbon group and preferably has a saturated hydrocarbon group.

The acyl group represented by A¹, A², and A³ may have 1 or more and 6 or less carbon atoms. In other words, cellulose acylate preferably has an acyl group having 1 or more and 6 or less carbon atoms.

The acyl group represented by A¹, A², and A³ may have a hydrogen atom substituted with a halogen atom (for example, a fluorine atom, a bromine atom, or an iodine atom), an oxygen atom, or a nitrogen atom, for example, but is preferably unsubstituted.

Examples of the acyl group represented by A¹, A², and A³ include a formyl group, an acetyl group, a propionyl group, a butyryl group (butanoyl group), a propenoyl group, and a hexanoyl group. Among these, acyl groups having 2 or more and 4 or less carbon atoms is preferable and an acyl group having 2 or 3 carbon atoms is more preferable as the acyl group from the viewpoint of improving the biodegradation speed.

Examples of the cellulose acylate include cellulose acetate (cellulose monoacetate, cellulose diacetate (DAC), and cellulose triacetate), cellulose acetate propionate (CAP), and cellulose acetate butyrate (CAB).

From the viewpoint of improving the rolling property, the oil absorbing property, and the mechanical strength, cellulose acylate may be cellulose acetate.

One cellulose acylate may be used alone or two or more cellulose acylates may be used in combination.

The weight-average polymerization degree of cellulose acylate is preferably 200 or more and 1000 or less, more preferably 500 or more and 1000 or less, and yet more preferably 600 or more and 1000 or less.

The weight-average polymerization degree of cellulose acylate is determined by the following procedure from the weight-average molecular weight (Mw).

First, the weight-average molecular weight (Mw) of cellulose acylate is measured on a polystyrene basis using tetrahydrofuran with a gel permeation chromatograph (GPC analyzer HLC-8320GPC produced by TOSOH CORPORATION, column: TSKgel α-M).

Next, the result is divided by the molecular weight of the constitutional unit of the cellulose acylate to determine the polymerization degree of cellulose acylate. For example, when the substituent of the cellulose acylate is an acetyl group, the molecular weight of the constitutional unit is 263 when the degree of substitution is 2.4 and is 284 when the degree of substitution is 2.9.

From the viewpoint of improving the rolling property, the oil absorbing property, and the mechanical strength, the degree of substitution of cellulose acylate is preferably 0.75 or less, more preferably 0.6 or less, and yet more preferably 0.2 or less.

The degree of substitution of cellulose acylate is an indicator of the extent in which the hydroxy groups in the cellulose are substituted with acyl groups. In other words, the degree of substitution is an indicator of the extent of acylation of cellulose acylate. Specifically, the degree of substitution means an intramolecular average of how many of the three hydroxy groups in a D-glucopyranose unit of cellulose acylate are substituted with acyl groups. The degree of substitution is determined from an infrared absorption spectrum (Spotlight 400, produced by PerkinElmer Inc.) through the absorbance ratio between the cellulose-derived peak (1030 cm⁻¹) and the acyl group-derived peak (1738 cm⁻¹).

Specifically, the absorbance at 1738 cm⁻¹/absorbance at 1030 cm⁻¹ ratio is assumed to be the degree of substitution.

### Polyester resin

Examples of the polyester resin include aliphatic polyester resins and aliphatic aromatic polyester resins.

Examples of the aliphatic polyester resins include polylactic acid (PLA), polyglycolic acid (PGA) polyhydroxybutyrate, poly(3-hydroxybutyrate-co-3-hydroxyhexanoate) (PHBH), polycaprolactone, polybutylene succinate (PBS), polybutylene succinate/adipate (PBSA), polyethylene succinate (PBA), and other polyhydroxyalkanoates.

Examples of the aliphatic aromatic polyester resins include polybutylene adipate-terephthalate copolymer resins (PBAH) and polytetramethylene adipate-terephthalate copolymer resins.

### Natural polymer

Examples of the natural polymer include starch, cellulose, chitin, chitosan, gluten, gelatin, zein, soy protein, collagen, and keratin.

### Other components

The resin particles of the exemplary embodiment may contain other components.

Examples of the other components include plasticizers, flame retardants, compatibilizers, release agents, light stabilizers, weather stabilizers, coloring agents, pigments, modifiers, anti-drip agents, antistatic agents, anti-hydrolysis agents, fillers, reinforcing agents (for example, glass fibers, carbon fibers, talc, clay, mica, glass flakes, milled glass, glass beads, crystalline silica, alumina, silicon nitride, aluminum nitride, and boron nitride), acid acceptors for preventing acetic acid release (oxides such as magnesium oxide and aluminum oxide, metal hydroxides such as magnesium hydroxide, calcium hydroxide, aluminum hydroxide, and hydrotalcite, calcium carbonate, and talc), and reactive trapping agents (for example, epoxy compounds, acid anhydride compounds, and carbodiimide).

The amount of the other components contained relative to the total amount of the resin particles (or base particles) may be 0 mass% or more and 5 mass% or less for each component. Here, "0 mass%" means that other components are not contained.

### Layer structure of resin particles

The resin particles of the exemplary embodiment may have a coating layer or no coating layer.

In other words, a resin particle of the exemplary embodiment may have a multilayer structure that includes a base particle containing a resin as a main component and a coating layer covering the base particle, or may have a single-layer structure that contains a resin as a main component. Here, a base particle is a particle onto which a coating layer is to be formed.

Alternatively, an intermediate layer may be interposed between the base particle and the coating layer.

When the resin particles of this exemplary embodiment do not require water repellency, the coating layer may be omitted, and when the resin particles of this exemplary embodiment require water repellency, a coating layer may be provided, and more preferably a coating layer having a coverage of 80% or more and 100% or less is provided.

### Coating layer

The coating layer may contain a coating material selected from hydrophobic compounds. Specifically, the coating layer may contain at least one coating material selected from the group consisting of fatty acids, fatty acid metal salts, and amino acid compounds.

### Fatty acids

Fatty acids are linear or branched saturated or unsaturated fatty acids. Fatty acids may be mixtures of saturated fatty acids and unsaturated fatty acids.

Fatty acids may have 14 or more and 22 or less (preferably 14 or more and 20 or less) carbon atoms. Specific examples of the linear fatty acids having 14 or more and 22 or less carbon atoms include behenic acid, arachidic acid, palmitic acid, stearic acid, isostearic acid, distearic acid, and myristic acid.

### Fatty acid metal salts

Fatty acid metal salts are linear or branched saturated or unsaturated fatty acid metal salts. Fatty acid metal salts may be mixtures of saturated fatty acid metal salts and unsaturated fatty acid metal salts.

Examples of the fatty acid metal salts include metal salts of fatty acids having 14 or more and 22 or less (preferably 14 or more and 20 or less) carbon atoms from the viewpoint of improving the water repellency against hot water and the property to maintain moist feel. Examples of the metal salts of fatty acids having 14 or more and 22 or less carbon atoms include metal salts of stearic acid, metal salts of behenic acid, and metal salts of palmitic acid, myristic acid, and distearic acid.

An example of the metal in the fatty acid metal salts is divalent metals.

Examples of the metal in fatty acid metal salts include magnesium, calcium, aluminum, barium, and zinc.

### Amino acid compounds

Amino acid compounds refer to amino acids and amino acid derivatives.

Examples of the amino acid compounds include lauroyl lysine, lauryl arginine, myristyl leucine, and stearoyl glutamic acid, and metal salts thereof.

The coating material constituting the coating layer is not limited to those coating materials described above, and other examples include coating materials such as lipid compounds (phospholipids, etc.), silicone compounds (dimethylsiloxane, hydrogen dimethicone, etc.), fluorinated compounds (perfluoropolyether, perfluorooctyltriethoxysilane, etc.), and ceramide compounds (hydroxypropylbispalmitamide MEA, etc.).

The coating amount of the coating layer relative to the base particle is preferably 1 mass% or more and 40 mass% or less, more preferably 3 mass% or more and 30 mass% or less, and yet more preferably 10 mass% or more and 30 mass% or less.

Here, the amount of the coating material contained relative to the entire coating layer is preferably 90 mass% or more and 100 mass% or less and more preferably 95 mass% or more and 100 mass% or less.

The coverage of the coating layer on the base particle is preferably 80% or more and 100% or less and more preferably 90% or more and 100% or less.

When the coverage of the coating layer is less than 80%, the regions where highly hydrophilic base particles are exposed increase, and the water resistance of the resin particles is degraded.

The coverage of the coating layer is measured as follows.

Resin particles to be measured are stained with ruthenium. Under scanning electron microscope (SEM) observation, the coating layer appears black by ruthenium staining.

The ruthenium-stained cellulose particles are observed with a SEM at a magnification of 3500x, and the ratio of the area of the observed black coating layer relative to the area of one resin particle observed is calculated.

This operation is carried out on 50 resin particles, and the arithmetic average of the obtained coating layer area ratios is calculated and assumed to be the coverage of the coating layer.

### Intermediate layer

The intermediate layer may contain at least one intermediate material selected from the group consisting of polyamine compounds, polyquaterniums, polysaccharide compounds, and polyacrylic acid.

Interposing the intermediate layer between the base particle and the coating layer inhibits separation of the coating layer.

A polyamine compound is a generic name for an aliphatic hydrocarbon having two primary amino groups.

Examples of the polyamine compound include polyalkyleneimine, polyallylamine, polyvinylamine, and polylysine.

From the viewpoint of improving the biodegradability, polyalkyleneimine preferably has a constitutional unit having an alkylene group having 1 or more and 6 or less carbon atoms (preferably 1 or more and 4 or less and more preferably 1 or more and 2 or less carbon atoms), and polyethyleneimine is more preferable.

Examples of the polyallylamine include homopolymers or copolymers of allylamine, allylamineamide sulfate, diallylamine, dimethylallylamine, etc.

Polyvinylamine is, for example, produced by hydrolysis of poly(N-vinylformamide) with an alkali, and a specific example thereof is "PVAM-0595B" produced by Mitsubishi Chemical Corporation.

Polylysine may be extracted from a natural product, produced by using transforming microorganisms, or synthesized chemically.

Examples of polyquaternium include polyquaternium-6, polyquaternium-7, polyquaternium-10, polyquaternium-11, polyquaternium-51, polyquaternium-61, and polyquaternium-64.

The amount of the polyquaternium contained relative to the total amount of the base particles may be 0.2 mass% or more and 2 mass% or less.

Examples of the polysaccharide compound include chitin, chitosan, and carboxymethylcellulose. Other examples of the polysaccharide compound include polysaccharides containing sulfuric acid or phosphoric acid, uronic acid (for example, glucuronic acid, iduronic acid, galacturonic acid, and mannuronic acid), or polysaccharides having both of these acid structures. Specific examples of the polysaccharides include hyaluronic acid, gellan gum, deacylated gellan gum (DAG), rhamsan gum, diutan gum, xanthan gum, carrageenan, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and alginic acid.

The intermediate material used in the intermediate layer is preferably a polysaccharide compound and more preferably chitosan.

The coating amount of the intermediate layer relative to the base particle is preferably 0.1 mass% or more and 20 mass% or less and more preferably 0.2 mass% or more and 2 mass% or less.

Here, the amount of the intermediate material contained relative to the entire intermediate layer is preferably 90 mass% or more and 100 mass% or less and more preferably 95 mass% or more and 100 mass% or less.

The ratio of the coating amount of the coating layer to the coating amount of the intermediate layer (in other words, ratio = coating amount of coating layer/coating amount of intermediate layer) is preferably 0.05 or more and 400 or less, more preferably 1.5 or more and 150 or less, and yet more preferably 5 or more and 150 or less.

### External additive

To the resin particles of the exemplary embodiment, inorganic particles may be added as an external additive. External addition of inorganic particles reduces secondary aggregation of the particles, and the particles easily exhibit their inherent properties.

The external additive is, for example, at least one selected from the group consisting of silicon-containing compound particles and metal oxide particles.

Silicon-containing compound particles refer to particles that contain silicon.

Silicon-containing compound particles may be particles solely composed of silicon or may be particles that contain silicon and other elements.

The silicon-containing compound particles may be silica particles. Silica particles may be any particles containing silica, that is, SiO₂, as a main component and may be crystalline or amorphous. The silica particles may be produced by using a silicon compound, such as water glass or alkoxysilane, as a raw material, and may be obtained by crushing quartz.

Oxides of metals other than silicon can be used as the metal oxide.

Examples of the metal oxide include zinc oxide, magnesium oxide, iron oxide, and aluminum oxide.

The volume-average particle size of the external additive is preferably 1 nm or more and 100 nm or less and more preferably 5 nm or more and 30 nm or less.

The volume-average particle size of the external additive is measured by the same method as measuring the volume-average particle size of the resin particles.

The amount of the external additive externally added relative to the entire resin particles (resin particles without any external additive externally added thereto) may be 0.1 mass% or more and 2 mass% or less.

### Method for producing resin particles

The method for producing the resin particles according to an exemplary embodiment is not particularly limited.

As a specific example, in order to obtain resin particles containing cellulose as a main component, the method for producing the resin particles according to an exemplary embodiment includes, for example, a step of forming cellulose acylate particles (hereinafter referred to as the particle forming step) and saponifying the cellulose acylate particles (hereinafter referred to as the saponifying step).

### Particle forming step

(1) First, cellulose acylate is dissolved in a water-soluble organic solvent A to prepare a cellulose acylate solution A.
(2) Next, the cellulose acylate solution A is added to a calcium carbonate dispersion containing calcium carbonate and a water-soluble inorganic salt dispersed in water, followed by stirring, to prepare a cellulose acylate liquid B.
(3) Next, the cellulose acylate liquid B is added to a mixed solution of carboxymethyl cellulose, a water-soluble organic solvent B, and water, and the resulting mixture is stirred to prepare a cellulose acylate liquid C.
(4) Next, the cellulose acylate liquid C is heated to remove the water-soluble organic solvents A and B, and sodium hydroxide and hydrochloric acid are added thereto to form cellulose acylate particles. Then the cellulose acylate particles are filtered out and dispersed in water to prepare a cellulose acylate particle dispersion D.

Here, the water-soluble organic solvents A and B are each a solvent in which 0.1 mass% or more and 10 mass% or less of water dissolves at 25°C relative to the solvent, and examples thereof include ethyl acetate, butyl acetate, methyl ethyl ketone, and acetone.

The water-soluble inorganic salt is preferably an inorganic salt that dissolves in an amount of 9% or more and more preferably in an amount of 15% or more in water at 20°C.

Examples of the water-soluble inorganic salt include sodium chloride, potassium chloride, lithium chloride, ammonium chloride, calcium chloride, magnesium chloride, sodium sulfate, potassium sulfate, ammonium sulfate, magnesium sulfate, sodium hydrogen sulfate, sodium nitrate, potassium nitrate, ammonium nitrate, calcium nitrate, magnesium nitrate, sodium carbonate, potassium carbonate, ammonium carbonate, and sodium hydrogen carbonate, among which a sodium salt, a potassium salt, and a magnesium salt are preferable, and sodium chloride, potassium chloride, magnesium chloride, sodium sulfate, potassium sulfate, and magnesium sulfate are particularly preferable.

The concentration of the water-soluble inorganic salt in the calcium carbonate dispersion relative to the dispersion is preferably 0.5 mass% or more and 20 mass% or less and more preferably 1 mass% or more and 10 mass% or less. At an excessively low concentration, formation of closed cells is inhibited, and at an excessively high concentration, it is difficult to obtain a satisfactory dispersion when the cellulose acylate solution A is mixed with the calcium carbonate dispersion.

### Saponifying step

(5) Next, sodium hydroxide is added to the cellulose acylate particle dispersion D, and then the resulting cellulose acylate particle dispersion D is heated in a weakly alkaline environment and stirred to saponify the cellulose acylate particles and to thereby prepare a cellulose particle suspension.
(6) Next, hydrochloric acid is added to the cellulose particle suspension to adjust the pH of the suspension to near neutral (for example, in the range of 6.5 or more and 7 or less), and then the cellulose particles are filtered out and washed with an organic solvent. Subsequently, the cellulose particles are repeatedly filtered out and washed with pure water. After the electrical conductivity of the filtrate has reached 10 µs/cm or less, the filtered out cellulose particles are dried.

Through the aforementioned steps, resin particles containing cellulose as a main component are obtained.

Here, in the method for producing the resin particles, when the cellulose acylate liquid B is added to the mixed solution of carboxymethyl cellulose, the water-soluble organic solvent B, and water and the resulting mixture is stirred to prepare cellulose acylate-containing oil droplets, water droplets are wrapped in the oil droplets. As a result, the water droplets are wrapped in the cellulose acylate particles formed. By drying these cellulose acylate particles, the water inside the particles evaporates, and closed cells are formed in the obtained resin particles.

Furthermore, the average long axis length of the closed cells and the closed cell porosity can be controlled by the type of the solvent, the concentration of the cellulose acylate solution A, the salt concentration of the cellulose acylate liquid B, and viscosity of the cellulose acylate liquid B, the oil phase-water phase interfacial tension, and the mechanical conditions such as stirring in preparing the cellulose acylate liquid C.

In order to obtain resin particles that have a volume-average particle size within the aforementioned range, for example, the type of solvent and the concentration of the cellulose acylate in the cellulose acylate solution A are adjusted.

Examples of the method for obtaining resin particles containing cellulose acylate as a main component include a method that involves obtaining cellulose acylate particles in the particle forming step without carrying out the saponifying step and a method involving decreasing the amount of sodium hydroxide in the saponifying step so as to decrease the extent of the saponification. The degree of substitution of cellulose acylate can be adjusted by decreasing the amount of sodium hydroxide in the saponifying step.

### Step of forming intermediate layer and step of forming coating layer

The method for producing resin particles according to the exemplary embodiment further includes an intermediate layer forming step and a coating layer forming step after the saponifying step when resin particles that have coating layers and intermediate layers are to be obtained. Specific details are as follows.

First, a water dispersion in which resin particles (hereinafter, referred to as "base particles") obtained through the saponifying step are dispersed is prepared. Before preparing the water dispersion, the base particles may be washed with an acid.

Next, the water dispersion containing the dispersed base particles is mixed with an aqueous solution containing an intermediate material constituting the intermediate layer. As a result, for example, the hydroxy groups in cellulose contained in the base particles react with carboxyl groups, amino groups, etc., of the intermediate material constituting the intermediate layer, and the hydroxy groups hydrogen-bond with each other to form an intermediate layer. When the intermediate layer is not to be formed, this operation is omitted.

Next, the water dispersion in which base particles with intermediate layers thereon are dispersed is heated, and then the coating material that constitutes coating layers are added thereto, followed by stirring. As a result, coating layers are formed.

In forming the coating layers, the coverage of the coating layer is controlled by adjusting the heating temperature, the amount of the coating material added, the time in which the coating material is added, and the stirring time after addition of the coating material.

Then resin particles with coating layers thereon are taken out from the resulting mixture. The resin particles with coating layers thereon are taken out by filtering the mixture, for example. The obtained resin particles with coating layers thereon may be washed with water. As a result, the unreacted coating material can be removed. Next, the resin particles with the coating layers thereon are dried to obtain resin particles according to an exemplary embodiment.

### External addition step

An external additive may be added to the obtained resin particles.

In the external addition step, for example, an external additive is added to the cellulose particles by using a mixing mill, a V blender, a Henschel mixer, a Lodige mixer, or the like.

### Usage

Examples of the usage of the resin particles according to an exemplary embodiment include particles for cosmetic preparations, paints, rolling agents, abrasives, scrubbing agents, display spacers, bead molding materials, light diffusing particles, resin reinforcing agents, refractive index controllers, biodegradation accelerators, fertilizers, water-absorbing particles, toner particles, and anti-blocking particles.

### Cosmetic preparation

A cosmetic preparation according to an exemplary embodiment contains the resin particles according to an exemplary embodiment, and examples thereof include skin cleansing agents or cosmetic preparations disclosed in Japanese Unexamined Patent Application Publication No. 2014-221743 or 2023-150333.

The form or phase of the cosmetic preparation according to this exemplary embodiment is not particularly limited, and examples thereof include powder, liquid, and solid.

The cosmetic preparation according to this exemplary embodiment may be any of a water-based, oil-based, or water-in-oil emulsion, an oil-in-water emulsion, nonaqueous emulsion, a W/O/W multi emulsion, and an O/W/O multi emulsion, for example.

Examples of the preparation forms of the cosmetic preparation according to this exemplary embodiment include liquid, cream, aerosol, ointment, emulsified solid, stick, and emulsified stick.

The usage of the cosmetic preparation according to this exemplary embodiment is also not particularly limited, and examples thereof include skincare cosmetics, scalp hair cosmetics, makeup cosmetics, UV protection cosmetics, antiperspirants, and cleansing agents.

Examples of the skincare cosmetics include lotions, milky lotions, creams, cleansing agents, oil liquids, hand creams, lip creams, and wrinkle concealing skincare cosmetics.

Examples of the scalp hair cosmetics include shampoos, conditioners, treatments, hair creams, cuticle coats, and setting agents.

Examples of the makeup cosmetics include makeup bases, concealers, face powders, powder foundations, liquid foundations, cream foundations, oil-based foundations, blushes, eye shadows, mascaras, eyeliners, eyebrow pencils, lipsticks, and nail products.

Examples of the UV protection cosmetics include sun-cut (sunscreen) oils, sun-cut milky lotions, sun-cut creams, sun-cut lotions, and sunscreens.

In particular, the cosmetic preparation of the present exemplary embodiment inhibits color darkening under sebum wetting conditions and has an excellent soft focus effect; thus, the cosmetic preparation is preferably for the makeup.

When the cellulose particles according to this exemplary embodiment are to be blended with the cosmetic preparation, the blend amount thereof is not particularly limited, and may be 0.01 mass% or more and less than 100 mass% relative to the cosmetic preparation. The preparation form, the usage, blending of optional components, and blend amounts of the optional components in the cosmetic preparation are not particularly limited, and may be any appropriate known preparation forms, usage, etc. The optional components are listed below. These optional components can be used alone or in combination.

### (1) UV absorber

The UV absorber may be any raw material that can usually be blended in a cosmetic preparation. Specific examples thereof include oxybenzone-1 (labeling name (INCI: Benzophenone-1)), oxybenzone-2 (labeling name (INCI: Benzophenone-2)), oxybenzone-3 (labeling name (INCI: Benzophenone-3)), oxybenzone-4 (labeling name (INCI: Benzophenone-4)), oxybenzone-5 (labeling name (INCI: Benzophenone-5)), oxybenzone-6 (labeling name (INCI: Benzophenone-6)), oxybenzone-9 (labeling name (INCI: Benzophenone-9)), homosalate, octocrylene, t-butylmethoxydibenzoylmethane, ethylhexyl salicylate, diethylamino hydroxybenzoyl hexyl benzoate, polysilicone-15, dimethoxybenzylidene dioxoimidazolidine octyl propionate (labeling name (INCI: Ethylhexyl Dimethoxybenzylidene Dioxoimidazolidine Propionate)), terephthalydene dicamphor sulfonic acid (labeling name (INCI: Terephthalylidene Dicamphor Sulfonic Acid)), ethylhexyl triazone, methylbis(trimethylsiloxy)silylisopentyl trimethoxycinnamate (labaling name (INCI: Isopentyl Trimethoxycinnamate Trisiloxane)), drometrizole trisiloxane, ethylhexyl dimethyl PABA (labeling name (INCI: Ethylhexyl Dimethyl PABA)), isopropyl paramethoxycinnamate (labeling name (INCI: Isopropyl Methoxycinnamate)), ethylhexyl methoxycinnamate, bisethylhexyloxyphenol methoxyphenyl triazine, phenylbenzimidazole sulfonic acid (labeling name (INCI: Phenylbenzimidazole Sulfonic Acid), methylenebisbenzotriazolyltetramethylbutylphenol, glyceryl dimethoxycinnamate ethylhexanoate (labeling name (INCI: Glyceryl Ethylhexanoate Dimethoxycinnamate)), glyceryl PABA, methyl diisopropylcinnamate (labeling name (INCI: Diisopropyl Methyl Cinnamate)), cinoxate, and ethylhexyl dimethoxybenzylidene dioxoimidazolidine propionate (labeling name (INCI: Ethylhexyl Dimethoxybenzylidene Dioxoimidazolidine Propionate)).

### (2) Oily material

The oily material may be solid, semisolid, or liquid, and, for example, natural animal and vegetable oils and fats, semi-synthetic oils and fats, hydrocarbon oils, higher fatty acids, higher alcohols, esters, silicone oils, and fluorine-based oily materials can be used.

### · Natural animal and vegetable oils and fats and semi-synthetic oils and fats

Examples of the natural animal and vegetable oily materials and semi-synthetic oily materials include natural vegetable oils such as avocado oil (labeling name (INCI: Persea Gratissima (Avocado) Oil)), linseed oil (labeling name (INCI: Linum Usitatissimum (Linseed) Seed Oil)), almond oil (labeling name (INCI: Prunus Amygdalus Dulcis (Sweet Almond)) Oil)), perilla oil, olive oil (labeling name (INCI: Olea Europaea (Olive) Fruit Oil)), Torreya californica oil (labeling name (INCI: Torreya Californica (California Nutmeg) Oil)), Cymbopogon nardus oil (INCI: Cymbopogon Nardus (Citronella) Oil)), Japanese torreya seed oil (labeling name (INCI: Torreya Nucifera Seed Oil)), apricot kernel oil (labeling name (INCI: Kyounin Yu)), wheat germ oil (labeling name (INCI: Triticum Vulgare (Wheat) Germ Oil)), sesame oil (labeling name (INCI: Sesamum Indicum (Sesame) Seed Oil)), wheat germ oil (labeling name (INCI: Triticum Vulgare (Wheat) Germ Oil)), rice germ oil (labeling name (INCI: Oryza Sativa (Rice) Germ Oil)), rice bran oil (labeling name (INCI: Oryza Sativa (Rice) Bran Oil)), sasanqua oil (labeling name (INCI: Camellia Kissi Seed Oil)), safflower oil (labeling name (INCI: Carthamus Tinctorius (Safflower) Seed Oil)), soybean oil (labeling name (INCI: Glycine Soja (Soybean) Oil)), tea seed oil (labeling name (INCI: Camellia Sinensis Seed Oil)), camellia oil (labeling name (INCI: Camellia Japonica Seed Oil)), evening primrose oil (labeling name (INCI: Oenothera Biennis (Evening Primrose) Oil)), rapeseed oil (labeling name (INCI: RAPE SHUSHI YU)), germ oil such as corn germ oil (labeling name (INCI: Zea Mays (Corn) Germ Oil)) and wheat germ oil (labeling name (INCI: Triticum Vulgare (Wheat) Germ) Oil), Prunus armeniaca oil (labeling name), palm oil (labeling name (INCI: Elaeis Guineensis (Palm) Oil)), palm kernel oil (labeling name (INCI: Elaeis Guineensis (Palm) Kernel Oil)), castor oil (labeling name (INCI: Ricinus Communis (Castor) Seed Oil)), sunflower oil (labeling name (INCI: Helianthus Annuus (Sunflower) Seed Oil)), grape seed oil (label name (INCI: Vitis Vinifera (Grape) Seed Oil)), jojoba seed oil (labeling name (INCI: Simmondsia Chinensis (Jojoba) Seed Oil)), macadamia seed oil (labeling name (INCI: Macadamia Ternifolia Seed Oil)), meadowfoam oil (labeling name (INCI: Limnanthes Alba (Meadowfoam) Seed oil), cottonseed oil (labeling name (INCI: Gossypium Herbaceum (Cotton) Seed Oil)), coconut oil (labeling name (INCI: Cocos Nucifera (Coconut) Oil)), and peanut oil (labeling name (INCI: Arachis Hypogaea (Peanut) Oil)), natural animal oil such as shark liver oil (labeling name (INCI: Shark Liver Oil)), cod liver oil (labeling name (INCI: Cod Liver Oil)), fish liver oil (labeling name (INCI: Fish Liver Oil)), turtle oil (labeling name (INCI: Turtle Oil)), mink oil (labeling name (INCI: Mink Oil)), and egg yolk oil (labeling name (INCI: Egg Oil)), and semi-synthetic oil such as hydrogenated coconut oil (labeling name (INCI: Hydrogenated Coconut Oil)) and liquid lanolin (labeling name (INCI: Lanolin Oil)).

### · Hydrocarbon oil

Examples of the hydrocarbon oil include olefin oligomers, isoparaffin such as C13-14 isoparaffin, and alkanes such as isododecane, undecane, dodecane, isohexadecane, hydrogenated polyisobutene (labeling name (INCI: Hydrogenated Polyisobutene)), squalane (INCI), mineral oil, coconut alkanes, C13-15 alkanes, and petrolatum (labeling name (INCI: Petrolatum)).

### · Higher fatty acid

Examples of the higher fatty acid include oleic acid (labeling name (INCI: Oleic Acid)), linoleic acid (labeling name (INCI: Linoleic Acid)), linolenic acid (labeling name (INCI: Linolenic Acid)), arachidonic acid (labeling name (INCI: Arachidonic Acid)), eicosapentaenoic acid (labeling name (INCI: Eicosapentaenoic Acid)), docosahexaenoic acid (labeling name (INCI: Docosahexaenoic Acid)), isostearic acid (labeling name (INCI: Isostearic Acid), and hydroxystearic acid (labeling name (INCI: Hydroxystearic Acid).

### · Higher alcohols

Examples of the higher alcohols include lauryl alcohol, myristyl alcohol, palmityl alcohol, stearyl alcohol, behenyl alcohol, oleyl alcohol, isostearyl alcohol, octyldodecanol, cholesterol, phytosterols, and batyl alcohol.

### · Ester oil

Examples of the ester oil include diisobutyl adipate (labeling name (INCI: Diisobutyl Adipate)), dihexyldecyl adipate (labeling name), diheptylundecyl adipate (labeling name (INCI: Diheptylundecyl Adipate)), n-alkyl glycol monoisostearates such as isostearyl isostearate (labeling name (INCI: Isostearyl Isostearate)), isocetyl isostearate (labeling name (INCI: Isocetyl Isostearate)), trimethylolpropane triisostearate (labeling name (INCI: Trimethylolpropane Triisostearate)), glycol diethylhexanoate (labeling name (INCI: Glycol Diethylhexanoate)), cetyl ethylhexanoate (labeling name (INCI: Cetyl Ethylhexanoate)), trimethylolpropane triethylhexanoate (labeling name (INCI: Trimethylolpropane Triethylhexanoate)), pentaerythrityl tetraethylhexanoate (labeling name (INCI: Pentaerythrityl Tetraethylhexanoate)), cetyl octanoate (labeling name (INCI: Cetyl Ethylhexanoate)), octyldodecyl esters such as octyldodecyl stearoyl oxystearate (labeling name (INCI: Octyldodecyl Stearoyl Stearate)), oleyl oleate (labeling name (INCI: Oleyl Oleate)), octyldodecyl oleate (labeling name (INCI: Octyldodecyl Oleate)), decyl oleate (labeling name (INCI: Decyl Oleate)), neopentyl glycol dioctanoate (labeling name (INCI: Neopentyl Glycol Diethylhexanoate)), neopentyl glycol dicaprate (labeling name (INCI: Neopentyl Glycol Dicaprate)), diisostearyl malate (labeling name (INCI: Diisostearyl Malate)), triethyl citrate (labeling name (INCI: Triethyl Citrate)), diethylhexyl succinate (labeling name (INCI: Diethylhexyl Succinate)), amyl acetate (labeling name (INCI: Amyl Acetate)), ethyl acetate (labeling name (INCI: Ethyl Acetate)), butyl acetate (labeling name (INCI: Butyl Acetate)), isocetyl stearate (labeling name (INCI: Isocetyl Stearate)), butyl stearate (labeling name (INCI: Butyl Stearate)), diisopropyl sebacate (labeling name (INCI: Diisopropyl Sebacate)), diethylhexyl sebacate (labeling name (INCI: Diethylhexyl Sebacate)), cetyl lactate (labeling name (INCI: Cetyl Lactate)), myristyl lactate (labeling name (INCI: Myristyl Lactate)), isononyl isononanoate (labeling name (INCI: Isononyl Isononanoate)), isotridecyl isononanoate (labeling name (INCI: Isotridecyl Isononanoate)), palmitic acid esters such as isopropyl palmitate (labeling name (INCI: Isopropyl Palmitate)), ethylhexyl palmitate (labeling name (INCI: Ethylhexyl Isopalmitate)), and hexyldecyl palmitate (labeling name (INCI: Isocetyl Palmitate, Hexyldecyl Palmitate)), cholesteryl hydroxystearate (labeling name (INCI: Cholesteryl Hydroxystearate)), myristic acid esters such as isopropyl myristate (labeling name (INCI: Isopropyl Myristate)), octyldodecyl myristate (labeling name (INCI: Octyldodecyl Myristate)), and myristyl myristate (labeling name (INCI: Myristyl Myristate)), ethylhexyl laurate (labeling name (INCI: Ethylhexyl Laurate)), hexyl laurate (labeling name (INCI: Hexyl Laurate)), dioctyldodecyl lauroyl glutamate (labeling name (INCI: Dioctyldodecyl Lauroyl Glutamate)), and isopropyl lauroyl sarcosinate (labeling name (INCI: Isopropyl Lauroyl Sarcosinate)). In addition, examples of the glyceride oil among the ester oil include triethylhexanoin (INCI), caprylic/capric triglyceride (labeling name (INCI: Caprylic/Capric Triglyceride)), cocoglyceryl (INCI), caprylic/capric/succinic triglyceride (labeling name (INCI: Caprylic/Capric/Succinic Triglyceride)), and caprylic/capric glycerides (labeling name (INCI: Caprylic/Capric Glycerides)).

### · Silicone oil

Examples of the silicone oil include alkyl-modified silicone such as dimethicone, trisiloxane, methyl trimethicone, ethyl trisiloxane, ethyl methicone, and hexyl dimethicone, long-chain alkyl-modified silicone such as caprylyl methicone, straight-chain or branched organopolysiloxane having low to high viscosity such as phenyl trimethicone, diphenyl dimethicone, diphenylsiloxy phenyl trimethicone, tetraphenyldimethyldisiloxane, and methylhydrogen polysiloxane, cyclic organopolysiloxane such as cyclotetrasiloxane, cyclopentasiloxane, and cyclohexasiloxane, amino-modified organopolysiloxane such as amodimethicone and aminopropyl dimethicone, pyrrolidone-modified organopolysiloxane and pyrrolidone carboxylic acid-modified organopolysiloxane such as PCA dimethicone, silicone rubber such as high-polymerization-degree gum-like dimethylpolysiloxane, gum-like amino-modified organopolysiloxane, and gum-like dimethylsiloxane·methylphenylsiloxane copolymer, low-viscosity organopolysiloxane solutions of silicone gum or rubber, and amino acid-modified silicone, fluorine-modified silicone, silicone resin, and a silicone resin dissolved matter.

### · Fluorine-based oily material

Examples of the fluorine-based oily material include perfluorodecalin, perfluorononyl dimethicone, and perfluoromethylcyclopentane.

### (3) Compounds having alcoholic hydroxyl groups

Examples of the compounds having alcoholic hydroxyl groups include ethanol (labeling name (INCI: Alcohol)), isopropanol (labeling name (INCI: Isopropyl Alcohol)), lower alcohols preferably having 2 to 5 carbon atoms, and sugar alcohols such as sorbitol, maltose, and xylitol. Other examples thereof include sterols such as cholesterol and sitosterol (labeling name (INCI: Beta-Sitosterol)), phytosterols, and lanosterol.

### (4) Surfactant

The surfactant can be nonionic, anionic, cationic, or amphoteric and may be any surfactant that is used in common cosmetics.

### (5) Powder

Examples of the powder include color pigments, inorganic powder, metal powder, organic powder, inorganic-organic hybrid powder. Specific details are as follows.

### · Color pigment

The color pigment may be any pigment that is used to color cosmetics, and examples thereof include red iron oxide (labeling name (INCI: Iron Oxides)), a yellow iron oxide (labeling name (INCI: Iron Oxides)), white titanium oxide (labeling name (INCI: Titanium Dioxide)), black iron oxide (labeling name (INCI: Iron Oxides)), ultramarine blue (labeling name (INCI: Ultramarines)), ferric hexacyanoferrate (labeling name (INCI: Ferric Ferrocyanide, Ferric Ammonium Ferrocyanide)), manganese violet (labeling name (INCI: Manganese Violet)), cobalt titanate (labeling name (INCI: Cobalt Titanium Oxide)), chromium hydroxide (labeling name (INCI: Chromium Hydroxide Green)), chromium oxide (labeling name (INCI: Chromium Oxide Greens)), (Al/cobalt) oxide (labeling name (INCI: Cobalt Aluminum Oxide)), cobalt titanate (labeling name (INCI: Cobalt Titanium Oxide)), (titanium/titanium oxide) calcinate (labeling name (INCI: Titanium/Titanium Dioxide)), (Li/cobalt) titanate (labeling name (INCI: Lithium Cobalt Titanate)), cobalt titanate (labeling name (INCI: Cobalt Titanium Oxide)), (iron oxide/titanium oxide) sinter (labeling name), composites doped with dissimilar metals such as iron oxide-doped titanium oxide (labeling name (INCI: Iron Oxides, Titanium Dioxide)), titanium nitride (labeling name (INCI: Titanium Nitride)), ferrous hydroxide (labeling name (INCI: Iron Hydroxide)), inorganic brown pigments such as γ-iron oxide, inorganic yellow pigments such as yellow ocher, and color pigments such as tar color lakes and natural color lakes. The shape of the pigment may be any, for example, may be spherical, substantially spherical, rod-like, spindle-like, petal-like, strip-like, or irregular, and the geometric form thereof is not particularly limited as long as color can be imparted to the cosmetics.

### · Inorganic powder

Examples of the inorganic powder include fine particles composed of zirconium oxide (labeling name (INCI: Zirconium Dioxide)), zinc oxide (labeling name (INCI: Zinc Oxide)), cerium oxide (labeling name (INCI: Cerium Oxide)), magnesium oxide (labeling name (INCI: Magnesium Oxide)), Ba sulfate (labeling name (INCI: Barium Sulfate)), calcium sulfate (labeling name (INCI: Calcium Sulfate), magnesium sulfate (labeling name (INCI: Magnesium Sulfate)), Ca carbonate (labeling name (INCI: Calcium Carbonate)), magnesium carbonate (labeling name (INCI: Magnesium Carbonate)), talc, mica, kaolin, synthetic fluorphlogopite (labeling name (INCI: Synthetic Fluorphlogopite), synthetic ferric gold mica, biotite (labeling name (INCI: Biotite), potassium silicate (labeling name (INCI: Potassium Silicate)), silica, aluminum silicate (labeling name (INCI: Aluminum Silicate)), magnesium silicate (labeling name (INCI: Magnesium Silicate)), aluminum/magnesium silicate (labeling name (INCI: Magnesium Aluminum Silicate)), calcium silicate (labeling name (INCI: Calcium Silicate)), aluminum calcium sodium silicate (labeling name (INCI: Aluminum Calcium Sodium Silicate)), lithium manganese sodium silicate (labeling name (INCI: Lithium Magnesium Sodium Silicate)), sodium manganese silicate (labeling name (INCI: Sodium Magnesium Silicate)), calcium aluminum borosilicate (labeling name (INCI: Calcium Aluminum Borosilicate)), calcium sodium borosilicate (labeling name (INCI: Calcium Sodium Borosilicate)), hydroxyapatite, bentonite, montmorillonite, hectorite, zeolite, aluminum, aluminum hydroxide (labeling name (INCI: Aluminum Hydroxide)), boron nitride (labeling name (INCI: Boron Nitride)), and glass (labeling name (INCI: Glass)). Furthermore, examples of the inorganic color pearl pigment include pearl agents such as mica coated with titanium oxide (labeling name (INCI: Titanium Dioxide)) and synthetic fluorophlogopite (labeling name (INCI: Synthetic Fluorphlogopite) coated with titanium oxide (labeling name (INCI: Titanium Dioxide)) and pearl pigments such as bismuth oxychloride (labeling name (INCI: Bismuth Oxychloride)), bismuth oxychloride (labeling name (INCI: Bismuth Oxychloride)) coated with titanium oxide (labeling name (INCI: Titanium Dioxide)), talc coated with titanium oxide (labeling name (INCI: Titanium Dioxide)), fish scale flakes (labeling name), and color mica coated with titanium oxide (labeling name (INCI: Titanium Dioxide)), and these may be untreated or may be surface-treated by a known method commonly employed in the cosmetic preparations.

### · Metal powder

Examples of the metal powder include metal fine particles composed of Al (labeling name (INCI: Aluminum, Aluminum Powder)), copper (labeling name (INCI: Copper Powder)), silver (labeling name (INCI: Silver Powder)), and gold (labeling name (INCI: Gold)).

### · Organic powder

Examples of the organic powder include powder composed of silicone, polyamide, polyacrylic acid·acrylic acid ester, polyester, polyethylene, polypropylene, polystyrene, styrene·acrylic acid copolymer, divinylbenzene·styrene copolymer, polyurethane, vinyl resin, urea resin, melamine resin, benzoguanamine, polymethylbenzoguanamine, tetrafluoroethylene, polymethylmethacrylate, cellulose, silk, nylon, phenolic resin, epoxy resin, and polycarbonate.

Other examples of the organic powder include organic colors, and specific examples thereof include tar colors such as Red 3, Red 104(1) (labeling name (INCI: Red 28, Red 28 Lake)), Red 106, Red 201 (labeling name (INCI: Red 6)), Red 202 (labeling name (INCI: Red 7)), Red 204, Red 205, Red 220 (labeling name (INCI: Red 34)), Red 226 (labeling name (INCI: Red 30)), Red 227 (labeling name (INCI: Red 33, RED 33 Lake)), Red 228 (labeling name (INCI: Red 36)), Red 230(1) (labeling name (INCI: Red 22, Red 22 Lake)), Red 230(2) (labeling name), Red 401, Red 505, Yellow 4 (labeling name (INCI: Yellow 5)), Yellow 5 (labeling name (INCI: Yellow 6, Yellow 6 Lake)), Yellow 202(1) (labeling name (INCI: Yellow 8)), Yellow 203 (labeling name (INCI: Yellow 10, Yellow 10 Lake)), Yellow 204 (labeling name (INCI: Yellow 11)), Yellow 401, Blue 1 (labeling name (INCI: Blue 1, Blue 1 Lake)), Blue 2, Blue 201, Blue 205 (labeling name (INCI: Blue 4)), Blue 404, Green 3 (labeling name (INCI: Green 3, Green 3 Lake)), Green 201 (labeling name (INCI: Green 5)), Green 202 (labeling name (INCI: Green 6)), Green 204 (labeling name (INCI: Green 8)), Green 205, Orange 201 (labeling name (INCI: Orange 5)), Orange 203 (labeling name (INCI: Pigment Orange 5)), Orange 204 (labeling name), Orange 205 (labeling name (INCI: Orange 4, Orange 4 Lake)), Orange 206 (labeling name (INCI: Orange 10)), and Orange 207 (labeling name (INCI: Orange 11)), and natural colors such as cochineal, laccaic acid (labeling name (INCI: Laccaic Acid)), safflower red (labeling name (INCI: Carthamus Tinctorius (Safflower) Flower Extract)), purple root extract (labeling name (INCI: Lithospermum Officinale Root Extract)), gardenia yellow, and gardenia blue (labeling name (INCI: Hydrolyzed Gardenia Florida Extract)).

### · Inorganic-organic hybrid powder

Examples of the inorganic-organic hybrid powder include hybrid powder in which surfaces of the inorganic powder are coated with organic powders by a known common method.

Note that the aforementioned powder may be a powder obtained by treating surfaces of particles. Here, any treatment agent for imparting hydrophobicity may be used, and examples thereof include silicone treatment agents, wax, paraffins, organofluorine compounds such as perfluoroalkyl-phosphate compounds, surfactants, amino acids such as N-acylglutamic acid, and metal soaps such as aluminum stearate and magnesium myristate.

### (6) Film forming agent

Examples of the film forming agent that can be used include trimethylsiloxysilicate, acrylic-silicone coating agents, silicone-modified norbornene, and silicone-modified pullulan.

### (7) Antiperspirant

The antiperspirant may be any component that suppresses sweat by contraction of skin, and a wide variety of common components can be used. Examples thereof include aluminum hydroxyhalides such as chlorohydroxyaluminum and aluminum chlorohydroxy allantoinate, aluminum halides such as aluminum chloride, allantoin aluminum salt, tannic acid, persimmon tannin, potassium aluminum sulfate, zinc oxide, zinc paraphenolsulfonate, burnt alum, aluminum zirconium tetrachlorohydrate, and aluminum zirconium trichlorohydrex GLY.

### (8) Antibacterial agent

Examples of the antibacterial agent that can be generally used include antibacterial chemicals such as triclosan, benzalkonium chloride, benzethonium chloride, chlorhexidine hydrochloride, chlorhexidine gluconate, halocarban, and isomethylphenol. Furthermore, natural medicine-derived essential oils and extracts that have antibacterial properties, such as Camellia sinensis leaf extract, may be added. Examples of the antibacterial agent having deodorizing effects such as natural medicine-derived essential oils and extracts include green tea extract, lavender extract, Scutellaria root extract, Coptis japonica extract, barley extract, Artemisia capillaris extract, aloe arborescens extract, Sophora arborescens root extract, Sasa veitchii leaf extract, garlic extract, Hamamelis virginiana extract, black tea extract, sage leaf extract, zanthoxylum pepper extract, ginger root extract, Calamus root extract, Hedera helix extract, Houttuynia cordata extract, peach fruit extract, peach leaf extract, peppermint leaf extract, Cnidium officinalis extract, eucalyptus leaf extract, peanut seed coat extract, lychee extract, and Sanguinea oleracea extract. The plant extracts may be used alone or in combination as a mixture.

### (9) Other additives

Examples of other additive include oil-soluble gelling agents, UV absorbing and scattering agents, moisturizing agents, preservatives, fragrances, salts, antioxidants, pH adjusters, chelating agents, cooling agents, anti-inflammatory agents, skin beautifying ingredients (skin whitening agents, cell activators, skin roughness improving agents, blood circulation promoters, skin astringents, antiseborrheic agents, etc.), vitamins, amino acids, nucleic acids, hormones, and inclusion compounds.

### · Oil-soluble gelling agent

Examples of the oil-soluble gelling agent include metal soaps such as aluminum stearate, magnesium stearate, and zinc myristate; amino acid derivatives such as lauroyl glutamic acid (labeling name (INCI: Lauroyl Glutamic Acid)) and α,γ-di-n-butylamine; dextrin fatty acid esters such as dextrin palmitate (labeling name (INCI: Dextrin Palmitate)), dextrin isostearate (labeling name (INCI: Dextrin Isostearate)), dextrin myristate (labeling name (INCI: Dextrin Myristate)), inulin stearate (labeling name (INCI: Stearoyl Inulin)), and dextrin palmitate/ethylhexanoate (labeling name (INCI: Dextrin Palmitate/Ethylhexanoate)); sucrose fatty acid esters such as sucrose palmitate and sucrose stearate; fructooligosaccharide fatty acid esters such as fructooligosaccharide stearate and fructooligosaccharide 2-ethylhexanoate; benzylidene derivatives of sorbitol such as monobenzylidene sorbitol and dibenzylidene sorbitol; organically modified clay minerals such as disteardimonium hectorite, stearalkonium hectorite, and hectorite; and stearalkonium bentonite.

### · UV absorbing and scattering agent

Examples of the UV absorbing and scattering agent that can be used include fine particle titanium oxide, fine particle iron-containing titanium oxide, fine particle zinc oxide, fine particle cerium oxide, hybrids thereof, and particles that absorb and scatter UV; and a dispersion prepared by dispersing UV-absorbing and scattering particles in an oily material in advance can also be used.

### · Moisturizing agent

Examples of the moisturizing agent include polyhydric alcohols such as BG (labeling name (INCI: Butylene Glycol)), PG (labeling name (INCI: Propylene Glycol)), DPG (labeling name (INCI: Dipropylene Glycol)), pentylene glycol, 1,10-decanediol, octanediol, 1,2-hexanediol, erythritol, glycerin, diglycerin, and polyethylene glycol; and glucose, glyceryl glucoside, betaine, sodium chondroitin sulfate (labeling name (INCI: Sodium Chondroitin Sulfate)), PCA-Na (labeling name (INCI: Sodium PCA)), methyl gluceth-10, methyl gluceth-20, hyaluronic acid, egg yolk lecithin, soy lecithin, phosphatidylcholine, phosphatidylethanolamine, phosphatidylserine, phosphatidylglycerol, phosphatidylinositol, and sphingolipids.

### · Preservative

Examples of the preservative include paraoxybenzoic acid alkyl ester, benzoic acid, sodium benzoate, sorbic acid, potassium sorbate, and phenoxyethanol, and examples of the antibacterial agent include benzoic acid, salicylic acid, carbolic acid, sorbic acid, paraoxybenzoic acid alkyl ester, parachlorometacresol, hexachlorophene, trichlorocarbanilide, photosensitizers, and phenoxyethanol.

### · Fragrance

There are natural fragrances and synthetic fragrances. Examples of the natural fragrance include plant-derived fragrances isolated from flowers, leaves, woods, fruit skin, etc.; and animal fragrances such as musk and civet. Examples of the synthetic fragrance include hydrocarbons such as monoterpene and alcohols such as aliphatic alcohols and aromatic alcohols; aldehydes such as terpene aldehyde and aromatic aldehyde; ketones such as alicyclic ketone; esters such as terpene esters; lactones; phenols; oxides; nitrogen-containing compounds; and acetals.

### · Salt

Examples of the salt include inorganic salts, organic acid salts, amine salts, and amino acid salts. Examples of the inorganic salt include sodium salts, potassium salts, magnesium salts, calcium salts, aluminum salts, zirconium salts, and zinc salts of inorganic acids such as hydrochloric acid, sulfuric acid, carbonic acid, and nitric acid; examples of the organic acid salts include salts of organic acids such as acetic acid, dehydroacetic acid, citric acid, malic acid, succinic acid, ascorbic acid, and stearic acid; and examples of the amine salts and amino acid salts include salts of amines such as triethanolamine and salts of amino acids such as glutamic acid. In addition, salts of hyaluronic acid, chondroitin sulfate, etc., aluminum zirconium glycine complexes, etc., and acid-alkali neutralization salts used in cosmetic formulations, etc., can also be used.

### · Antioxidant

The antioxidant may be any antioxidant, and examples thereof include carotenoids, ascorbic acid and salts thereof, ascorbyl stearate, tocophenol, tocophenol acetate, tocopherol, p-t-butylphenol, butylhydroxyanisole, dibutylhydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, campherol, myricetin, and quercetin. These antioxidants may be used alone or in combination.

### · pH adjuster

Examples of the pH adjuster include lactic acid, citric acid, glycolic acid, succinic acid, tartaric acid, dl-malic acid, potassium carbonate, sodium bicarbonate, and ammonium bicarbonate.

### · Chelating agent

Examples of the chelating agent include alanine, edetic acid sodium salt, sodium polyphosphate, sodium metaphosphate, and phosphoric acid.

### · Cooling agent

Examples of the cooling agent include L-menthol and camphor.

### · Anti-inflammatory agent

Examples of the anti-inflammatory agent include allantoin, glycyrrhizinic acid and its salts, glycyrrhetinic acid and stearyl glycyrrhetinate, tranexamic acid, and azulene.

### · Skin beautifying ingredient

Examples of the skin beautifying ingredient include cellular stimulants such as placenta extract, arbutin, glutathione, saxifrage extract and other skin whitening agents, royal jelly, photosensitizers, cholesterol derivatives, and calf blood extract; blood circulation promoting agents such as skin roughness soothing agents, nonylic acid vanillylamide, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthin, and γ-oryzanol, skin astringents such as zinc oxide and tannic acid, and antiseborrheic agents such as sulfur and thianthrol.

### · Vitamins

Examples of the vitamins include vitamin A such as vitamin A oil, retinol, retinol acetate, and retinol palmitate; vitamin B such as vitamin B2 such as riboflavin, riboflavin butyrate, and flavin adenine nucleotide, vitamin B6 such as pyridoxine hydrochloride, pyridoxine dioctanoate, and pyridoxine tripalmitate, vitamin B12 and derivatives thereof, and vitamin B15 and derivatives thereof; vitamin C such as L-ascorbic acid, L-ascorbic acid dipalmitate, L-ascorbic acid-2-sodium sulfate, and dipotassium L-ascorbate phosphate diester; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and nicotinamide; vitamin H; vitamin P; pantothenic acids such as calcium pantothenate, D-pantothenyl alcohol, pantothenyl ethyl ether, and acetyl pantothenyl ethyl ether; and biotins.

### · Amino acids

Examples of the amino acid include glycine, valine, leucine, isoleucine, serine, threonine, phenylalanine, arginine, lysine, aspartic acid, glutamic acid, cystine, cysteine, methionine, and tryptophan.

### · Nucleic acid

An example of the nucleic acid is deoxyribo nucleic acid.

### · Hormone

Examples of the hormone include estradiol and ethenyl estradiol.

### · Inclusion compound

An example of the inclusion compound is cyclodextrin.

### EXAMPLES

Examples, which do not limit the scope of the present disclosure, will now be described. In the description below, "parts" and "%" are all on a mass basis unless otherwise noted.

### Example 1

### Production of resin particles CP 1

### Particle forming step

In 870 parts of ethyl acetate, 130 parts of cellulose acylate ("L-50" produced by Daicel Corporation) is completely dissolved. The resulting solution is added to a liquid prepared by dispersing 60 parts of calcium carbonate and 26 parts of sodium sulfate in 600 parts of water, and the resulting mixture is stirred for 1 hour. The resulting dispersion is added to a solution prepared by dispersing 3.0 parts of carboxymethyl cellulose (hereinafter may also be referred to as "CMC") and 120 parts of ethyl acetate in 600 parts of pure water.

The resulting mixture is stirred in a high-speed emulsifier (T25 digital ULTRA-TURRAX produced by IKA)) at a rotation rate of 8500 min⁻¹ for 10 minutes. Thereto, 10 parts of sodium hydroxide is added, followed by stirring at 80°C for 3 hours to remove the organic solvent.

To the obtained liquid, 10 parts of diluted hydrochloric acid is added, and the residue is filtered and then is dispersed again in pure water to obtain a cellulose acylate particle dispersion (solid concentration: 10%).

### Saponifying step

To 500 parts of the obtained cellulose acylate particle dispersion, 17.5 parts of a 20% aqueous sodium hydroxide solution is added, followed by stirring for 6 hours at a saponifying temperature of 30°C. After hydrochloric acid is added to the saponified suspension to adjust the pH to 7, the obtained cellulose particles are filtered out and repeatedly washed with pure water, and the cellulose particle cake obtained by washing until the electrical conductivity of the filtrate is 50 µs/cm or less is again prepared into a slurry with pure water to obtain a cellulose particle dispersion (solid concentration: 20%).

### Surface treatment

To 500 parts of the cellulose particle dispersion, 1 part of chitosan lactate is added, followed by stirring at room temperature for 5 hours, then 8 parts of sodium stearate is added thereto as a fatty acid salt, and the resulting mixture is heated to 75°C. Thereto, 65 parts of a 5% aqueous calcium chloride solution is added as an aqueous salt solution, and the resulting mixture is stirred for 20 minutes to form coating layers. Next, filtration and washing are repeated on the obtained particles until the electrical conductivity of the filtrate is 50 µs/cm or less. After washing, the obtained cake is dried to obtain cellulose particles having coating layers. The obtained particles are stirred in an FM mixer (FM40 produced by NIPPON COKE & ENGINEERING. CO., LTD.) by maintaining the mixer temperature at 25°C at a rotation rate of 2000 min⁻¹ for 1 hour to dress the surfaces of the coating layers.

Through the aforementioned steps, resin particles are obtained.

### Example 2

### Production of resin particles CP2

Resin particles CP2 are obtained as with the resin particles CP1 except that the number of parts of CMC is changed as indicated in Table 1 during the particle forming step of the resin particles CP1.

### Example 3

### Production of resin particles CP3

Resin particles CP3 are obtained as with the resin particles CP1 except that the saponifying step performed in producing the resin particles CP1 is omitted.

### Examples 4 to 11

### Production of resin particles CP4 to CP11

Resin particles CP4 to CP11 are obtained as with the resin particles CP1 except that the following matters are changed as indicated in Table 1 during the particle forming step of the resin particles CP1.
· The number of parts of ethyl acetate in which cellulose acylate is dissolved.
· In the case where a solvent other than ethyl acetate is used to dissolve cellulose acylate, this solvent is mixed with ethyl acetate and used.
· The number of parts of CMC.
· When a surfactant is to be used, the surfactant is added simultaneously with the CMC.
· The type and amount of the aqueous salt solution are changed.

### Example 12

### Production of resin particles CP12

Resin particles CP12 not subjected to surface treatment are obtained by drying the cake, which is obtained at the last stage of the saponifying step, in the particle forming step of the resin particles CP1.

### Example 13

### Production of resin particles CP13

Resin particles CP13 are obtained by performing the same process up to the saponifying step in the particle forming step of the resin particles CP1, and then performing different surface treatment as below.

### Surface treatment

To 500 parts of the cellulose particle dispersion, 1 part of chitosan lactate is added, followed by stirring at room temperature for 5 hours. After the pH is adjusted to 6 by adding an aqueous sodium hydroxide solution, the particles are repeatedly filtered and washed until the electrical conductivity of the filtrate is 50 µs/cm or less. After washing, the obtained cake is dried to obtain particles CP13.

### Comparative Examples 1 and 4

### Production of resin particles CPC1 to CPC4

Resin particles CPC1 to CPC4 are obtained as with the resin particles CP1 except that the following matters are changed as indicated in Table 1 during the particle forming step of the resin particles CP1.
· The number of parts of ethyl acetate in which cellulose acylate is dissolved.
· In the case where a solvent other than ethyl acetate is used to dissolve cellulose acylate, this solvent is mixed with ethyl acetate and used.
· The number of parts of sodium sulfate.
· The number of parts of CMC.
· When a surfactant is to be used, the surfactant is added simultaneously with the CMC.
· Stirring conditions used for the high-speed emulsifier in the particle forming step.

### Comparative Example 5

Porous Cellulose particles ("CELLULOBEADS D-10" produced by DAITO KASEI KOGYO CO., LTD.) are prepared.

### Comparative Example 6

Nylon particles ("SP-10" produced by TORAY INDUSTRIES, INC.) are prepared.

### Comparative Example 7

Titanium oxide particles ("Ti-pure R104" produced by The Chemours Company) are prepared.

### Comparative Example 8

Porous cellulose particles ("CELLUFLOW C-25" produced by JNR Corporation) are prepared.

### Evaluation

Particles of each example are evaluated as follows.

### Properties

The properties of the particles of each example are measured by the aforementioned methods.
Volume-average particle size of particles (particle D50v)
Average long axis length of closed cells
Closed cell porosity
Open cell porosity
Average circularity of particles

### Shield maintaining properties in a wet state

The bottom of a disk-shaped aluminum container (area: about 8.5 cm²) for producing foundations is painted red.

Next, 1 g of particles of each example is placed on the bottom of the container and put under a pressure of 100 kg/cm² to solidify. Next, the solidified solid film is wetted by adding two drops of artificial sebum (ARTIFICIAL SEBUM "D4265-14" produced by BIOCHEMAZONE).

Then the degree of transparency and the degree of red observed in the wet portion are visually examined by the following standard.
A: The wet portion does not turn transparent and no red color is observed.
B: The wet portion turns slightly transparent but no red color is observed.
C: The wet portion turns transparent but no red color is observed.
D: The wet portion turns transparent and red color is observed.

### Light transmitting properties and haze

Into a 20 mL vial, 0.5 g of particles of each example and 4.5 g of a liquid silicone film-forming agent ("KF-7312L" produced by Shin-Etsu Chemical Co., Ltd.) are placed, and the resulting mixture is stirred with a rotor for 24 hours and then ultrasonically treated for 2 minutes. The prepared mixture is formed into a 30 µm-thick film with an applicator on a PET film, and the film is dried overnight to prepare a film for evaluation.

The film for evaluation is analyzed with a haze meter to measure the total transmittance and the diffuse transmittance. Then the haze value is calculated from the equation: haze value = diffuse transmittance/total transmittance × 100. The evaluation standard is as follows.
Total transmittance
   A: 86% or more.
   B: 83% or more and less than 86%.
   C: 76% or more and less than 83%.
   D: Less than 76%.
Haze
   A: 80% or more.
   B: 75% or more and less than 80%.
   C: 68% or more and less than 75%.
   D: Less than 75%.

### Biodegradability

Four-week aerobic biodegradation rate of particles of each example is measured in accordance with JIS K 6950:2000 (ISO 14851:1999). The evaluation standard is as follows.
A: Degradation rate is 60% or more.
B: Degradation rate is 30% or more and less than 60%.
C: Degradation rate is less than 30%.

The abbreviations used in Tables 1 and 2 are as follows.
SANa: sodium stearate
SGNa: sodium stearoyl glutamate
SA/Ca: calcium salt of stearic acid
SA/Al: aluminum salt of stearic acid
SA/Mg: magnesium salt of stearic acid
SG/Ca: calcium salt of stearoyl glutamic acid
SG/Al: aluminum salt of stearoyl glutamic acid
Tween 85: Polyoxyethylene (20) sorbitan trioleate (Tokyo Chemical Industry Co., Ltd.)

**Table 1**

| | Particles | Particle main component | Ethyl acetate Number of parts | Other solvents /Number of parts | Sodium sulfate Number of parts | CMC Number of parts | Surfactant /Number of parts | High-speed emulsifier stirring condition [rpm] | Fatty acid salt | Aqueous salt solution /Number of parts |
|---|---|---|---|---|---|---|---|---|---|---|
| Example 1 | CP1 | Cellulose | 870 | - | 26 | 3.0 | - | 8500 | SANa | Calcium chloride/65 |
| Example 2 | CP2 | Cellulose | 870 | - | 26 | 3.2 | - | 8500 | SANa | Calcium chloride/65 |
| Example 3 | CP3 | DAC | 870 | - | 26 | 3.0 | - | 8500 | SANa | Calcium chloride/65 |
| Example 4 | CP4 | Cellulose | 740 | Chloroform/130 | 45 | 3.0 | - | 8500 | SANa | Calcium chloride/65 |
| Example 5 | CP5 | Cellulose | 670 | MEK/200 | 26 | 3.0 | - | 8500 | SANa | Calcium chloride/65 |
| Example 6 | CP6 | Cellulose | 610 | Chloroform/260 | 46 | 3.0 | - | 8500 | SANa | Calcium chloride/65 |
| Example 7 | CP7 | Cellulose | 870 | - | 26 | 3.0 | Tween85/1 | 8500 | SANa | Calcium chloride/65 |
| Example 8 | CP8 | Cellulose | 870 | - | 26 | 3.0 | - | 8500 | SANa | Aluminum chloride /92 |
| Example 9 | CP9 | Cellulose | 870 | - | 26 | 3.0 | - | 8500 | SGNa | Aluminum chloride /92 |
| Example 10 | CP10 | Cellulose | 870 | - | 26 | 3.0 | - | 8500 | SGNa | Calcium chloride/65 |
| Example 11 | CP11 | Cellulose | 870 | - | 26 | 3.0 | - | 8500 | SANa | Magnesium chloride/90 |
| Example 12 | CP12 | Cellulose | 870 | - | 26 | 3.0 | - | 8500 | No coating layer | |
| Example 13 | CP13 | Cellulose | 870 | - | 26 | 3.0 | - | 8500 | Coated with chitosan only | |
| Comparative Example 1 | CPC1 | Cellulose | 570 | MEK/300 | 3 | 3.0 | - | 8500 | SANa | Calcium chloride/65 |
| Comparative Example 2 | CPC2 | Cellulose | 870 | - | 26 | 1.5 | - | 4000 | SANa | Calcium chloride/65 |
| Comparative Example 3 | CPC3 | Cellulose | 870 | - | 26 | 3.0 | Tween85/1.5 | 8500 | SANa | Calcium chloride/65 |
| Comparative Example 4 | CPC4 | Cellulose | 300 | Chloroform/570 | 50 | 3.2 | - | 8500 | SANa | Calcium chloride/65 |

**Table 2**

| | Particles | Particle main component | Coating layer | D50v | Closed cell average long axis length | Closed cell porosity | Open cell porosity | Average circularity | Shield maintaining properties | Total transmittance | Haze | Biodegradability |
|---|---|---|---|---|---|---|---|---|---|---|---|---|
| | | | | µm | µm | % | % | - | | | | |
| Example 1 | CP1 | Cellulose | SA/Ca | 8 | 0.3 | 7 | 0 | 0.96 | A | A | A | A |
| Example 2 | CP2 | Cellulose | SA/Ca | 7 | 0.5 | 8 | 0 | 0.96 | A | A | A | A |
| Example 3 | CP3 | DAC | SA/Ca | 7 | 0.6 | 8 | 0 | 0.96 | A | A | B | C |
| Example 4 | CP4 | Cellulose | SA/Ca | 7 | 0.9 | 14 | 0 | 0.94 | A | A | B | A |
| Example 5 | CP5 | Cellulose | SA/Ca | 9 | 0.6 | 1 | 0 | 0.96 | B | A | B | A |
| Example 6 | CP6 | Cellulose | SA/Ca | 7 | 0.6 | 18 | 0 | 0.96 | A | B | B | A |
| Example 7 | CP7 | Cellulose | SA/Ca | 8 | 1.4 | 8 | 0 | 0.96 | A | A | B | A |
| Example 8 | CP8 | Cellulose | SA/AI | 8 | 0.3 | 7 | 0 | 0.96 | A | A | A | A |
| Example 9 | CP9 | Cellulose | SG/Al | 8 | 0.3 | 7 | 0 | 0.96 | A | A | A | A |
| Example 10 | CP10 | Cellulose | SG/Ca | 8 | 0.3 | 7 | 0 | 0.96 | A | A | A | A |
| Example 11 | CP11 | Cellulose | SA/Mg | 8 | 0.3 | 7 | 0 | 0.96 | A | A | A | A |
| Example 12 | CP12 | Cellulose | No coating | 8 | 0.3 | 7 | 0 | 0.96 | A | A | A | A |
| Example 13 | CP13 | Cellulose | Only chitosan | 8 | 0.3 | 7 | 0 | 0.96 | A | A | A | A |
| Comparative Example 1 | CPC1 | Cellulose | SA/Ca | 12 | 0.3 | 0.2 | 0 | 0.95 | D | A | C | A |
| Comparative Example 2 | CPC2 | Cellulose | SA/Ca | 34 | 0.5 | 8 | 0 | 0.94 | A | D | D | A |
| Comparative Example 3 | CPC3 | Cellulose | SA/Ca | 8 | 2.1 | 8 | 0 | 0.96 | A | A | C | A |
| Comparative Example 4 | CPC4 | Cellulose | SA/Ca | 8 | 0.5 | 42 | 10 | 0.96 | A | D | C | A |
| Comparative Example 5 | CELLULOBEADS D-10 | Cellulose | - | 14 | - | 0 | 0 | 0.94 | D | A | D | A |
| Comparative Example 6 | SP-10 | Nylon | - | 7 | - | 0 | 0 | 0.96 | C | A | C | C |
| Comparative Example 7 | Ti-pure R104 | Titanium oxide | - | 0.2 | - | 0 | 0 | 0.96 | Impossible to evaluate | D | A | C |
| Comparative Example 8 | CELLUFLOW C-25 | Cellulose | - | 9 | 1.8 | 60 | Difficult to determine | 0.91 | C | D | B | C |

The aforementioned results indicate that the resin particles of Examples exhibit excellent shield maintaining properties in a wet state compared to the particles of Comparative Examples. In addition, both the transmitting properties and haze can be achieved.

This indicates that, when the resin particles of Examples are used in cosmetic preparations, color darkening caused by sebum wetting is reduced, and a cosmetic preparation offers an excellent soft focus effect.

### Product evaluation

The cosmetic preparation evaluation is performed as follows by using the resin particles CP1 of Example 1 or the resin particles CP12 of Example 12.

### W/O sunscreen

### Composition of W/O sunscreen

· Ingredient 1: Resin particles CP1 of Example 1: 1.0 part
· Ingredient 2: Decamethylcyclopentasiloxane (note 1): 12.0 parts
· Ingredient 3: Diphenylsiloxyphenyl trimethicone (note 2): 2.0 parts · Ingredient 4: Ethylhexyl methoxycinnamate: 7.5 parts
· Ingredient 5: Diethylamino hydroxybenzoyl hexyl benzoate: 2.0 parts · Ingredient 6: Orthotrichlene: 2.5 parts
· Ingredient 7: Crosslinked polyether-modified silicone mixture (note 3): 4.0 parts · Ingredient 8: Cetyl PEG/PPG-10/1 dimethicone (note 4): 2.0 parts
· Ingredient 9: 1,3-Butylene glycol: 3.0 parts
· Ingredient 10: Sodium citrate: 0.5 parts
· Ingredient 11: Sodium chloride: 0.5 parts
· Ingredient 12: Ethanol: 5.0 parts
· Ingredient 13: Purified water: 58.0 parts

(note 1) KF-995 (product of Shin-Etsu Chemical Co., Ltd.)
(note 2) KF-56A (product of Shin-Etsu Chemical Co., Ltd.)
(note 3) KSG-210 (product of Shin-Etsu Chemical Co., Ltd.)
(note 3) KF-6048 cetyl PEG/PPG-10/1 dimethicone (product of Shin-Etsu Chemical Co., Ltd.)

### Production and evaluation of W/O sunscreen

The aforementioned ingredients 1 to 8 are stirred and mixed to homogeneity. Separately, ingredients 9 to 12 are homogeneously dissolved in ingredient 13, and a mixture of the aforementioned ingredients 1 to 8 are moderately added, followed by stirring, to obtain an emulsion. The resulting emulsion is filled in a predetermined container to obtain a W/O sunscreen.

The obtained W/O sunscreen is light to the touch without causing oily sensation, spreads smoothly, has high transparency, and forms a highly water-repellent even film.

### O/W sunscreen cream

### Composition of O/W sunscreen cream

· Ingredient 1: Polyglyceryl-5 stearate (note 1): 5.0 parts
· Ingredient 2: Cetanol: 2.0 parts
· Ingredient 3: Butylene glycol: 3.0 parts
· Ingredient 4: Triethylhexanoin: 7.5 parts
· Ingredient 5: Polyglyceryl-10 decaisostearate (note 2): 0.5 parts
· Ingredient 6: Cyclopentasiloxane: 5.0 parts
· Ingredient 7: Mineral oil: 7.5 parts
· Ingredient 8: Cetyl ethylhexanoate: 7.5 parts
· Ingredient 9: Lipophilic fine particle titanium oxide: 5.0 parts
· Ingredient 10: Lipophilic fine particle zinc oxide: 1.0 part
· Ingredient 11: Resin particles CP 1 of Example 1: 1.0 part
· Ingredient 12: Glyceryl behenate, polyglyceryl-6 octastearate (note 3): 0.5 parts
· Ingredient 13: Xanthan gum: 0.2 parts
· Ingredient 14: Bentonite (10% water swelling solution): 5.0 parts
· Ingredient 15: water: 49.3 parts

(note 1) SUNSOFT A-181E-C (product of Taiyo Kagaku Co., Ltd.)
(note 2) SUNOIL DDI (product of Taiyo Kagaku Co., Ltd.)
(note 3) TAISET 50-C (product of Taiyo Kagaku Co., Ltd.)

### Production/evaluation of O/W sunscreen cream

· Procedure A: Ingredient 1 and ingredient 15 are mixed, ingredient 2 and part of ingredient 3 are added thereto, and the resulting mixture is mixed.
· Procedure B: Ingredients 4 to 8 are mixed, and ingredients 9 to 11 and ingredient 12 are added thereto under stirring.
· Procedure C: Under stirring, the mixture obtained in the procedure B is gradually added to the mixture obtained in the procedure A, followed by stirring.
· Procedure D: The rest of ingredient 3 and ingredient 13 are added and dispersed, and ingredient 14 is mixed.
· Procedure E: Under stirring, the mixture obtained in the procedure D is gradually added to the mixture obtained in the procedure C, and the resulting mixture is stirred to obtain an O/W sunscreen cream.

The obtained O/W sunscreen cream spreads lightly, is fresh without stickiness, has high transparency, and forms an even film.

### Lipstick

### Composition of lipstick

· Ingredient 1: Decamethylcyclopentasiloxane: 28.7 parts
· Ingredient 2: Isotridecyl isononanoate: 10.0 parts
· Ingredient 3: Diisocytearyl malate: 5.0 parts
· Ingredient 4: Candelilla wax: 10.0 parts
· Ingredient 5: Polyethylene: 6.0 parts
· Ingredient 6: Resin particles CP1 of Example 1: 1.0 part
· Ingredient 7: Microcrystalline wax: 2.0 parts
· Ingredient 8: Trimethylsiloxysilicate mixture (note 1): 20.0 parts
· Ingredient 9: (Vinyl dimethicone/lauryl dimethicone) crosspolymer (note 2): 5.0 parts
· Ingredient 10: Silicone branched alkyl polyglycerin co-modified silicone (note 3): 3.0 parts
· Ingredient 11: Colorant: 1.3 parts
· Ingredient 12: mica: 8.0 parts

(note 1) KF-7312J: a 50%/50% mixture of trimethylsiloxysilicate and cyclopentasiloxane (Shin-Etsu Chemical Co., Ltd.)
(note 2) KSG-43: a mixture of (vinyl dimethicone/lauryl dimethicone) crosspolymer + triethylhexanoin (product of Shin-Etsu Chemical Co., Ltd.)
(note 3) KF-6115: lauryl polyglyceryl-3 polydimethylsiloxyethyl dimethicone (product of Shin-Etsu Chemical Co., Ltd.)

### Production/evaluation of lipstick

· Procedure A: Ingredient 11 is mixed with part of ingredient 2 and is dispersed in a roll mill, and the resulting dispersion is heated and mixed together with ingredient 1, the rest of ingredient 2, and ingredients 3 to 12 to obtain a composition.
· Procedure B: The composition obtained in the procedure A is poured into a mold, and is cooled and solidified to obtain a lipstick.

The obtained lipstick is found to spread smoothly and form an excellent even film.

### Non-aqueous mousse foundation

### Composition of non-aqueous mousse foundation

· Ingredient 1: (Dimethicone/(PEG-10/15)) crosspolymer (note 1): 18.00 parts
· Ingredient 2: Dimethicone (note 2): 1.00 part
· Ingredient 3: Decamethylcyclopentasiloxane: 11.00 parts
· Ingredient 4: Ethylhexyl methoxycinnamate: 5.00 parts
· Ingredient 5: Jojoba oil: 1.00 part
· Ingredient 6: Silylated silicic anhydride (note 3): 0.75 parts
· Ingredient 7: KTP-09R (note 4): 0.20 parts
· Ingredient 8: KTP-09Y (note 4): 1.00 part
· Ingredient 9: KTP-09B (note 4): 0.02 parts
· Ingredient 10: KTP-09W (note 4): 5.00 parts
· Ingredient 11: KF-9909 (note 5) treated talc: 11.55 parts
· Ingredient 12: Trimethylsiloxysilicate dissolved product (note 6): 4.00 parts
· Ingredient 13: Decamethylcyclopentasiloxane (note 7): 25.28 parts
· Ingredient 14: Polysilicone-1 crosspolymer (note 8): 6.00 parts
· Ingredient 15: Polymethylsilsesquioxane (note 9): 3.00 parts
· Ingredient 16: Resin particles CP1 of Example 1: 7.00 parts
· Ingredient 17: Antioxidant: 0.20 parts

(note 1) KSG-240: a mixture of cyclopentasiloxane + (dimethicone/(PEG-10/15)) crosspolymer
(product of Shin-Etsu Chemical Co., Ltd.)
(note 2) KF-96A (product of Shin-Etsu Chemical Co., Ltd.)
(note 3) Surface-hydrophobized fumed silica: AEROSIL R-972 (product of NIPPON AEROSIL CO., LTD.)
(note 4) KF-9909-treated color inorganic pigment, W: white, R: red, Y: yellow, B: black (Shin-Etsu Chemical Co., Ltd.)
(note 5) Triethoxysilylethyl polydimethylsiloxyethyl hexyl dimethicone (product of Shin-Etsu Chemical Co., Ltd.)
(note 6) KF-7312J: 50% trimethylsiloxysilicate dissolved in cyclopentasiloxane (product of Shin-Etsu Chemical Co., Ltd.)
(note 7) KF-995 (product of Shin-Etsu Chemical Co., Ltd.)
(note 8) KSP-411 (product of Shin-Etsu Chemical Co., Ltd.)
(note 9) KMP-590 (product of Shin-Etsu Chemical Co., Ltd.)

### Production/evaluation of non-aqueous mousse foundation

Ingredients 1 to 10 are mixed to homogeneity by roller treatment. To the resulting mixture, ingredients 11 to 17 are mixed to homogeneity to obtain a non-aqueous mousse foundation.

The obtained foundation appears to have a firm mousse form, spreads lightly without stickiness or oiliness, has excellent feel in use, and is confirmed to have excellent lasting effects.

### Rinse-off cosmetic pack

### Composition of rinse-off cosmetic pack

· Ingredient 1: Dimethicone (note 1): 3.0 parts
· Ingredient 2: Decamethylcyclopentasiloxane: 3.0 parts
· Ingredient 3: Polyglycerin-modified silicone oil (note 2): 2.0 parts
· Ingredient 4: Kaolin: 28.0 parts
· Ingredient 5: Resin particles CP12 of Example 12: 2.0 parts
· Ingredient 6: Carbomer: 0.4 parts
· Ingredient 7: Butylene glycol: 10.0 parts
· Ingredient 8: Glycerin: 20.0 parts
· Ingredient 9: Preservative: 0.1 parts
· Ingredient 10: Fragrance: 0.1 parts
· Ingredient 11: water: 31.4 parts

(note 1) KF-96A (product of Shin-Etsu Chemical Co., Ltd.)
(note 2) KF-6105 polyglyceryl-3 polydimethylsiloxyethyl dimethicone (product of Shin-Etsu Chemical Co., Ltd.)

### Production/evaluation of rinse-off cosmetic pack

· Procedure A: Ingredients 1 to 3 and 9 are mixed.
· Procedure B: Ingredients 6 to 8 and 11 are mixed to homogeneity, and then ingredients 4, 5, and 10 are mixed and stirred.
· Procedure C: To the mixture obtained in the procedure B, the mixture obtained in the procedure A is added and emulsified to obtain a paste-like rinse-off cosmetic pack.

The obtained rinse-off cosmetic pack spreads lightly during application, offers excellent washing effects, and leaves skin moist and smooth without stickiness after the rinsing; thus, this rinse-off cosmetic pack is found to have excellent feel in use and excellent stability.

### Deodorant stick

### Composition of deodorant stick

· Ingredient 1: Chlorohydroxy Al: 23.00 parts
· Ingredient 2: Decamethylcyclopentasiloxane: 36.50 parts
· Ingredient 3: Stearyl alcohol: 8.00 parts
· Ingredient 4: Talc: 13.88 parts
· Ingredient 5: Fragrance: 0.10 parts
· Ingredient 6: BHT (dibutylhydroxytoluene): 0.02 parts
· Ingredient 7: Paraffin (solid): 2.00 parts
· Ingredient 8: Mineral oil: 14.5 parts
· Ingredient 9: Crosslinked polyether-modified silicone mixture (note 1): 1.00 part
· Ingredient 10: Resin particles CP1 of Example 1: 1.00 part
   (note 1) KF-6048 cetyl PEG/PPG-10/1 dimethicone (product of Shin-Etsu Chemical Co., Ltd.)

### Production/evaluation of deodorant stick

· Procedure A: Ingredients 2 and 3 and 6 to 9 are heated, dissolved, and mixed.
· Procedure B: To the mixture obtained in the procedure A, ingredients 1, 4, and 10 are added and mixed to homogeneity, and the resulting mixture is dispersed and mixed to homogeneity by using a mixer.
· Procedure C: To the mixture obtained in the procedure B, ingredient 5 is added and mixed to homogeneity.
· Procedure D: The mixture obtained in the procedure C is poured into a mold, and is cooled and solidified.

The obtained deodorant stick does not have excessive dry feel or stickiness and has highly lasting deodorizing effects.

### Skin cleansing agent

### Composition of skin cleansing agent

· Ingredient 1: Resin particles CP1 of Example 1: 1.0 part
· Ingredient 2: Polyoctanium-7 (note 1): 0.1 parts
· Ingredient 3: Potassium myristate: 20.0 parts
· Ingredient 4: Potassium palmitate: 5.0 parts
· Ingredient 5: Potassium stearate: 5.0 parts
· Ingredient 6: Potassium laurate: 3.0 parts
· Ingredient 7: Glycerin: 25.0 parts
· Ingredient 8: PEG-6 (H(OCH₂CH₂)ₙOH (n = average 6)): 5.0 parts
· Ingredient 9: PEG-32 (H(OCH₂CH₂)ₙOH (n = average 32)): 5.0 parts
· Ingredient 10: Sorbitol: 5.0 parts
· Ingredient 11: Glycosyl trehalose (note 2): 2.0 parts
· Ingredient 12: Glyceryl stearate: 1.5 parts
· Ingredient 13: Kaolin: 1.0 parts
· Ingredient 14: Phytosteryl isostearate: 0.5 parts
· Ingredient 15: Sodium methyl cocoyl taurate: 1.0 part
· Ingredient 16: Hydrolyzed hydrogenated starch: 1.2 parts
· Ingredient 17: Water: 18.7 parts

(note 1) MERQUAT 550 (product of The Lubrizol Corporation)
(note 2) Tornare (product of Hayashibara Co., Ltd.)

### Production/evaluation of skin cleansing agent

Ingredients 1 to 17 described above are mixed by using a homomixer to prepare a skin cleansing agent.

When a face is washed with cleansing foam made from the obtained skin cleansing agent, the washed face is refreshed and has appropriate moist feel.

### Powder foundation

### Composition of powder foundation

**Table 3**

| Powder foundation | | | |
|---|---|---|---|
| Formulation | Compound | Product name (manufacturer) | Parts by mass |
| Particles | Particles | Resin particles CP1 of Example 1 | 10 |
| Powders other than the aforementioned particles | Talc | Talc CT-25 (YAMAGUCHI MICA CO., LTD.) | 50 |
| | Mica | Mica FA450 (YAMAGUCHI MICA CO., LTD.) | 19 |
| | Titanium oxide | MKR-1 (SAKAI CHEMICAL INDUSTRY CO., LTD.) | 9.5 |
| | Black iron oxide | C33-134 Sun CROMA Black Iron Oxide (Sun Chemical) | 0.2 |
| | Red iron oxide | C33-128 Sun CROMA Red Iron Oxide (Sun Chemical) | 0.4 |
| | Yellow iron oxide | C33-210 Sun CROMA Yellow Iron Oxide (Sun Chemical) | 2.4 |
| Binder | Diisostearyl malate | Neosolue-DiSM (Nippon Fine Chemical Co., Ltd.) | 3 |
| | Caprylic/capric triglyceride | Caprylic/capric triglyceride (FUJIFILM Wako Pure Chemical Corporation) | 2 |
| | Neopentyl glycol dicaprate | NPDC (KOKYU ALCOHOL KOGYO CO., LTD.) | 2 |
| | Pentylene glycol | Diol PD (KOKYU ALCOHOL KOGYO CO., LTD.) | 1.5 |
| Total | | | 100 |

### Production/evaluation of powder foundation

Ingredients indicated in Table 3 above are compacted by using a metal dish press to obtain a powder foundation.

When the obtained powder foundation is applied to the inner side of an arm by using a powder puff, the applied foundation is smooth and provided moist feel.

The foregoing description of the exemplary embodiments of the present disclosure has been provided for the purposes of illustration and description. It is not intended to be exhaustive or to limit the disclosure to the precise forms disclosed. Obviously, many modifications and variations will be apparent to practitioners skilled in the art. The embodiments were chosen and described in order to best explain the principles of the disclosure and its practical applications, thereby enabling others skilled in the art to understand the disclosure for various embodiments and with the various modifications as are suited to the particular use contemplated. It is intended that the scope of the disclosure be defined by the following claims and their equivalents.

### Appendix

(((1))) Resin particles having closed cells inside, wherein the resin particles have a volume-average particle size of 1 µm or more and 30 µm or less, the closed cells have an average long axis length of 0.1 µm or more and 2.0 µm or less, and a closed cell porosity is 1% or more and 40% or less.
(((2))) The resin particles described in (((1))), comprising a biodegradable resin as a main component.
(((3))) The resin particles described in (((2))), wherein the biodegradable resin is a cellulose.
(((4))) The resin particles described in any one of (((1))) to (((3))), wherein the closed cells have an average long axis length of 0.1 µm or more and 1.5 µm or less.
(((5))) The resin particles described in any one of (((1))) to (((4))), wherein the closed cell porosity is 1% or more and 20% or less.
(((6))) A cosmetic preparation comprising the resin particles described in any one of (((1))) to (5).

The effects of the foregoing are as follows.

According to (((1))), there are provided particles that have closed cells inside, that have excellent shield maintaining properties in a wet state, and that can achieve both transmitting properties and haze compared to when the volume-average particle size is less than 1 µm or more than 30 µm, the average long axis length of the closed cells is less than 0.1 µm or more than 2.0 µm, or the closed cell porosity is less than 1% or more than 40%.

According to (((2))), there are provided resin particles that have excellent biodegradability and shield maintaining properties in a wet state and that can achieve both transmitting properties and haze compared to when a non-biodegradable resin is contained as a main component.

According to (((3))), there are provided resin particles that have excellent biodegradability and shield maintaining properties in a wet state and that can achieve both transmitting properties and haze compared to when a cellulose derivative is contained.

According to (((4))), there are provided resin particles that have excellent shield maintaining properties in a wet state and that can achieve both transmitting properties and haze compared to when the closed cells have an average long axis length of less than 0.1 µm or more than 1.5 µm.

According to (((5))), there are provided resin particles that have excellent shield maintaining properties in a wet state and that can achieve both transmitting properties and haze compared to when the closed cell porosity is less than 1% or more than 20%.

According to (((6))), there is provided a cosmetic preparation that reduces color darkening caused by sebum wetting and exhibits an excellent soft focus effect compared to when resin particles that have closed cells inside, that have a volume-average particle size of less than 1 µm or more than 30 µm, that have a closed cell average long axis length of less than 0.1 µm or more than 2.0 µm, and that have a closed cell porosity of less than 1% or more than 40% are used.

## Claims

1. Resin particles having closed cells inside,
wherein the resin particles have a volume-average particle size of 1 µm or more and 30 µm or less,
the closed cells have an average long axis length of 0.1 µm or more and 2.0 µm or less, and
a closed cell porosity is 1% or more and 40% or less.

2. The resin particles according to claim 1, comprising a biodegradable resin as a main component.

3. The resin particles according to claim 2, wherein the biodegradable resin is a cellulose.

4. The resin particles according to any one of claims 1 to 3, wherein the closed cells have an average long axis length of 0.1 µm or more and 1.5 µm or less.

5. The resin particles according to any one of claims 1 to 4, wherein the closed cell porosity is 1% or more and 20% or less.

6. A cosmetic preparation comprising the resin particles according to any one of claims 1 to 5.
